(19) Europäisches Patentamt
European Patent Office
Office européen des brevets

(11) EP 3 287 453 A1

(12) **EUROPEAN PATENT APPLICATION**
published in accordance with Art. 153(4) EPC

(43) Date of publication:
**28.02.2018 Bulletin 2018/09**

(21) Application number: **16782661.9**

(22) Date of filing: **22.04.2016**

(51) Int Cl.:
*C07D 413/10* (2006.01) *C07D 233/60* (2006.01)
*C07D 413/06* (2006.01) *C07D 413/08* (2006.01)
*C07D 413/14* (2006.01) *C07D 417/10* (2006.01)
*C07D 401/10* (2006.01) *A61K 31/422* (2006.01)
*A61P 9/10* (2006.01)

(86) International application number:
**PCT/CN2016/079992**

(87) International publication number:
**WO 2016/169514 (27.10.2016 Gazette 2016/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.04.2015 CN 201510200711**

(71) Applicant: **Medshine Discovery Inc.**
**Nanjing, Jiangsu 210032 (CN)**

(72) Inventors:
- **LONG, Chaofeng**
**Dongguan City**
**Guangdong 523325 (CN)**
- **CHEN, Shuhui**
**Shanghai 200131 (CN)**
- **CHEN, Xiaoxin**
**Dongguan City**
**Guangdong 523325 (CN)**
- **LUO, Yunfu**
**Shanghai 200131 (CN)**
- **LIU, Zhuowei**
**Dongguan City**
**Guangdong 523325 (CN)**
- **PAN, Jianfeng**
**Shanghai 200131 (CN)**

(74) Representative: **Haseltine Lake LLP**
**Redcliff Quay**
**120 Redcliff Street**
**Bristol BS1 6HU (GB)**

(54) **IMIDAZOLE COMPOUND**

(57) Disclosed is an imidazole compound, in particular, the compound as shown in formula (I) and a pharmaceutically acceptable salt or tautomer thereof are disclosed.

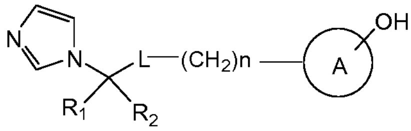

(I)

EP 3 287 453 A1

## Description

CROSS REFERENCE TO RELATED PATENT APPLICATIONS

This application claims priority to CN Patent Application CN201510200711.8, filed April 24, 2015, the contents of which are incorporated herein by reference in their entireties.

TECHNICAL FIELD

The present invention relates to an imidazole compound, specifically relates to a compound represented by formula (I), a pharmaceutically acceptable salt or tautomer thereof.

BACKGROUND OF THE PRESENT INVENTION

Ischemic cerebrovascular disease (ischemic stroke) is due to the temporary or permanent reduction of blood supply to an arterial area caused by embolism or bleeding, its pathological process involves complicated time and spatial cascade reaction, and the mechanism is related to $Ca^{2+}$ overload, free radical damage and other factors. Ozagrel Sodium shows a specific inhibitory action on thromboxane-A synthase, resulting in pharmacological effects such as anti-platelet aggregation and dilation of blood vessels. In addition, Ozagrel exhibits the ability to reduce the generation of free radicals through the inhibition of lipid peroxidation, and to remove directly free radicals leading to improved tolerance of brain tissue to hypoxia conditions. It was widely used in the treatment of ischemic cerebrovascular diseases including acute cerebral infarction, and achieved significant effect.

Ozagrel was developed as a novel anti-platelet drug by Ono Pharmaceutical Co. Ltd. in the Japan. In 1989, it was introduced to the market (Cataclot®) for the first specific thromboxane synthase A2 (TXA2) inhibitor. It inhibits the conversion of prostaglandin H2 (PGH2) derived from platelet into thromboxane A2 (TXA2), and thereby promotes the generation of prostaglandin PGI2, which was synthesized from PGH2 in endothelial cell. By way of improving the balance between TXA2 and PGI2, the drug achieves the therapeutic effect of ischemic cerebrovascular disease.

The structure of Ozagrel is as follows:

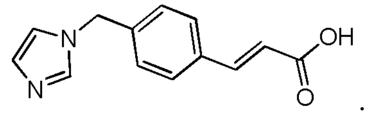

CONTENTS OF THE PRESENT INVENTION

The aim of the present invention is to provide a compound of formula (I), a pharmaceutically acceptable salt or a tautomer thereof,

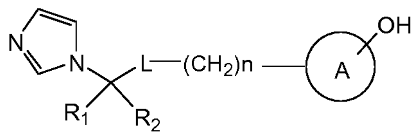

( I )

wherein, n is an integer of 0 to 3, preferably 0 or 1; L is selected from a 5- to 6- membered cyclohydrocarbyl or heterocyclohydrocarbyl or $-(CH_2)_{1-6}-$, each of which is optionally substituted by R;

ring A is selected from a 5- to 6- membered unsaturated cyclohydrocarbyl or heterocyclyl, each of which is optionally substituted by R

each of $R_1$, $R_2$, R is independently selected from H, F, Cl, Br, I, CN, OH, SH, $NH_2$, CHO, COOH, or selected from $C(=O)NH_2$, $S(=O)NH_2$, $S(=O)_2NH_2$, an $C_{1-6}$ alkyl or heteroalkyl, a $C_{3-6}$ cycloalkyl or heterocycloalkyl, each of which is optionally substituted by $R_{01}$;

"hetero-" represents a heteroatom or a heteroatomic group, which is selected from - C(=O)N(R)-, -N(R)-, -C(=NR)-, $-S(=O)_2N(R)-$, -S(=O)N(R)-, -O-, -S-, -C(=O)O-, -C(=O)-, -C(=S)-, -S(=O)-, $-S(=O)_2-$, or -N(R)C(=O)N(R)-;

each of the number of $R_{01}$, the heteroatom or the heteroatomic group is independently selected from 0, 1, 2 or 3; and

$R_{01}$ is selected from H, F, Cl, Br, I, CN, OH, an $C_{1-3}$ alkyl, $N(CH_3)_2$, $NH(CH_3)$, $NH_2$, CHO, COOH, $C(=O)NH_2$, $S(=O)NH_2$, $S(=O)_2NH_2$, trifluoromethyl, aminomethyl, hydroxymethyl, methoxyl, formoxyl, methoxycarbonyl, methylsulfonyl, methylsulfinyl.

In one embodiment of the present invention, each of the above-mentioned $R_1$, $R_2$, R is independently selected from H, F, Cl, Br, I, an $C_{1-3}$ alkyl, an $C_{1-3}$ alkoxy, $N(CH_3)_2$, $NH(CH_3)$, $NH_2$, CHO, COOH, $C(=O)NH_2$, $S(=O)NH_2$, $S(=O)_2NH_2$, trifluoromethyl, aminomethyl, hydroxymethyl, formoxyl, methoxycarbonyl, methylsulfonyl, methylsulfinyl, cyclopropyl.

In one embodiment of the present invention, each of the above-mentioned $R_1$, $R_2$, R is independently selected from H, F, Cl, Br, I, $CH_3$, $C_2H_5$-, $CH_3O$-, or

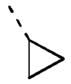.

In one embodiment of the present invention, the above-mentioned L is selected from a 5- to 6-membered aryl or heteroaryl, a 5- to 6- membered aliphatic cyclohydrocarbyl, $-(CH_2)_{1-6}-$, each of which is optionally substituted by R.

In one embodiment of the present invention, the above-mentioned L is selected from

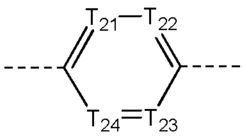, 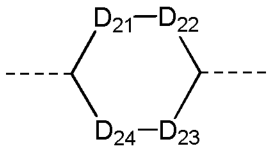, 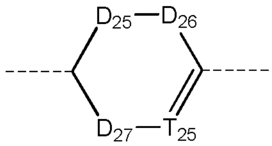

or $-(CH_2)_{1-6}-$ which is optionally substituted by R, wherein,
none or one of $T_{21-24}$ is N, and the others are C(R);
zero to three of $D_{21-24}$ are selected from -C(=O)N(R)-, -N(R)-, -C(=NR)-, $-S(=O)_2N(R)-$, - S(=O)N(R)-, -O-, -S-, -C(=O)O-, -C(=O)-, -C(=S)-, -S(=O)-, $-S(=O)_2-$ or -N(R)C(=O)N(R)-, and the others are C(R) (R);
$T_{25}$ is N or C(R);
zero to three of $D_{25-27}$ are selected from -C(=O)N(R)-, -N(R)-, -C(=NR)-, $-S(=O)_2N(R)-$, - S(=O)N(R)-, -O-, -S-, -C(=O)O-, -C(=O)-, -C(=S)-, -S(=O)-, $-S(=O)_2-$ or -N(R)C(=O)N(R)-, and the others are C(R) (R).

In one embodiment of the present invention, the above-mentioned L is selected from

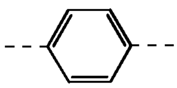, 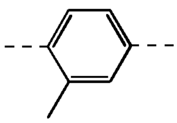, 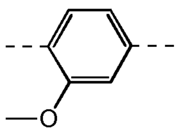, 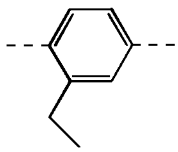, 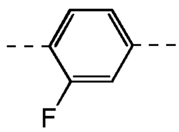,

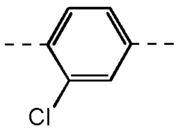, 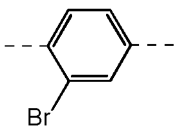, 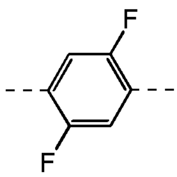, 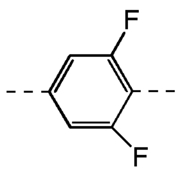, 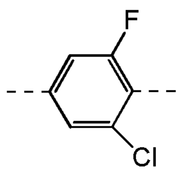,

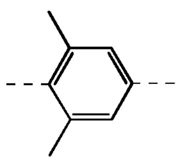, 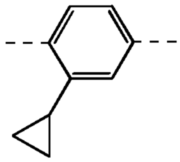, 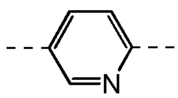, 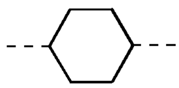, 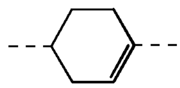,

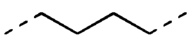.

In one embodiment of the present invention, the above-mentioned A is selected from a 5- to 6-membered aryl or heteroaryl.

In one embodiment of the present invention, the above-mentioned A is selected from

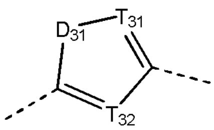 , 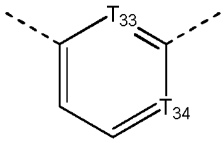 ;

each of $T_{31-34}$ is independently selected from N or C(R),
$D_{31}$ is selected from -C(R)(R)-, -C(=O)N(R)-, -N(R)-, -C(=NR)-, $-S(=O)_2N(R)-$, -S(=O)N(R)-, -O-, -S-, -C(=O)O-, -C(=O)-, -C(=S)-, -S(=O)-, $-S(=O)_2-$ or -N(R)C(=O)N(R)-.

In one embodiment of the present invention, the above-mentioned A is selected from

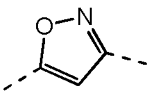 , 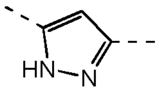 , 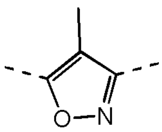 , 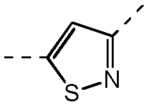 , 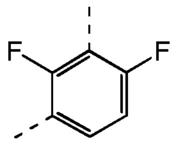 , 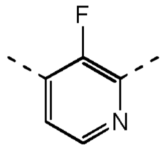 .

In one embodiment of the present invention, the above-mentioned moiety

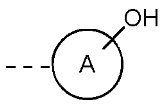

is selected from

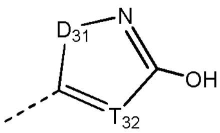 , 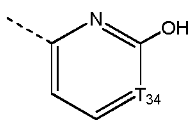 , 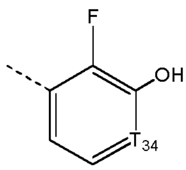 ;

the tautomer thereof is selected from

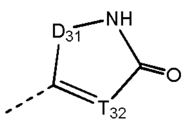 , 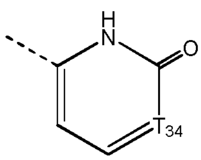 .

In one embodiment of the present invention, the above-mentioned moiety

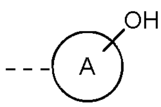

is selected from the group consisting of

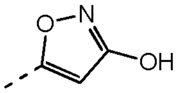 , 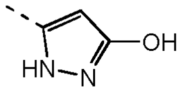 , 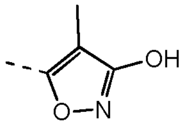 ,

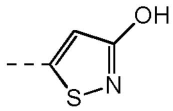 , 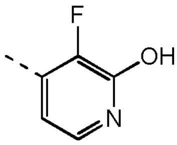 , 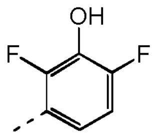 ;

the tautomer thereof is selected from the group consisting of

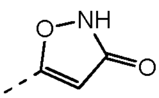 , 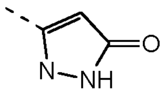 , 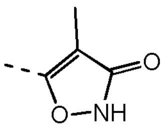 , 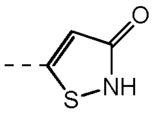 , 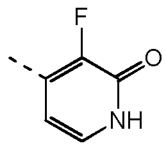 .

In one embodiment of the present invention, the above-mentioned compound, the pharmaceutically acceptable salt or the tautomer thereof, is selected from the group consisting of

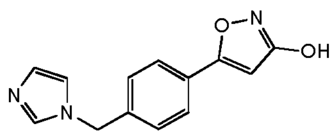 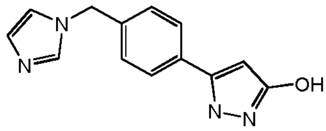 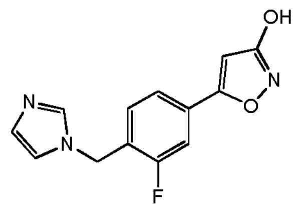

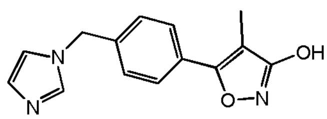 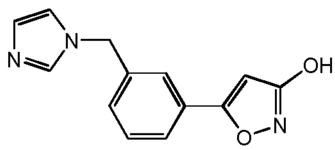 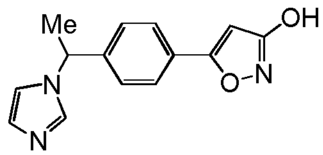

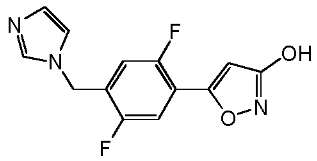 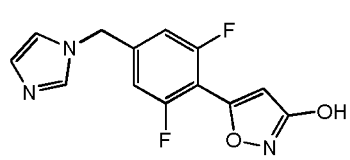 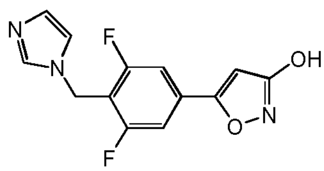

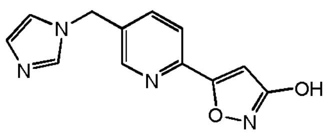 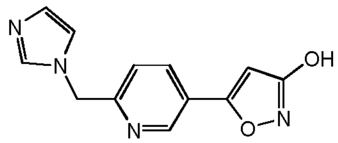 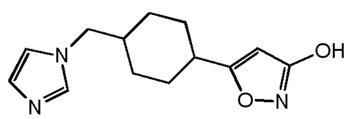

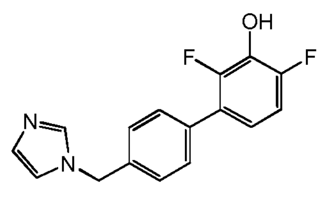 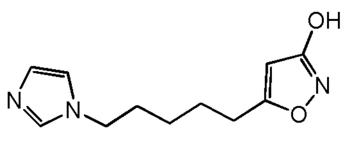 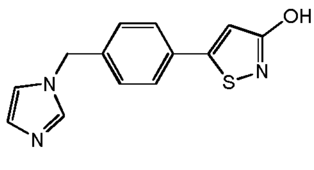

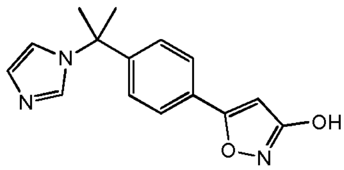 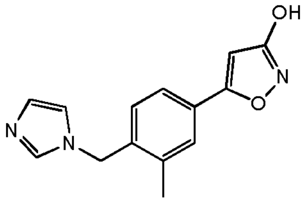 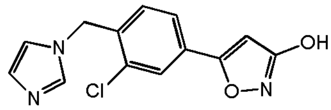

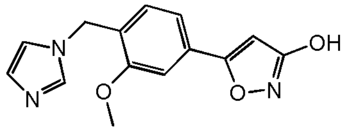 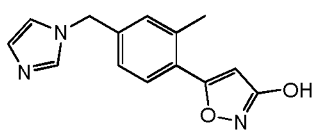 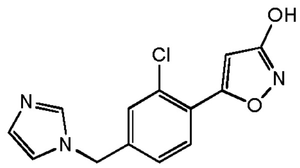

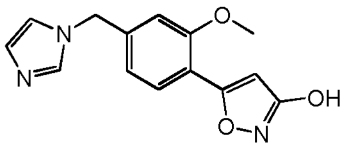 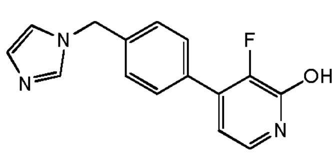 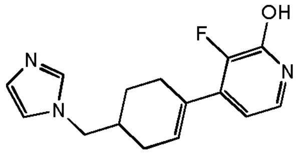

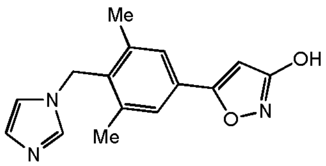 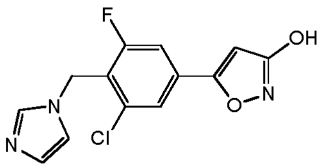 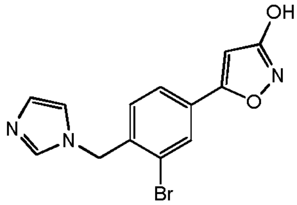

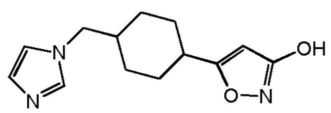 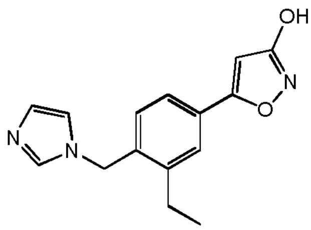 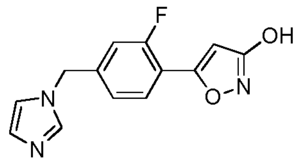

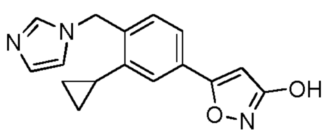 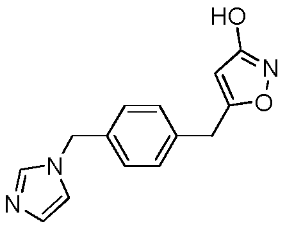 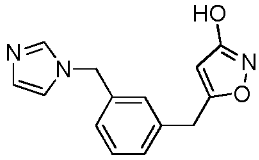

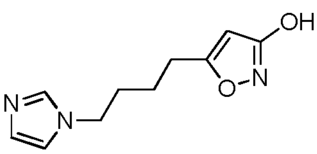 .

In one embodiment of the present invention, the above-mentioned compound, the pharmaceutically acceptable salt or the tautomer thereof, is selected from the group consisting of

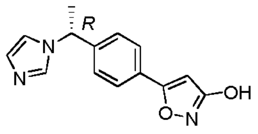 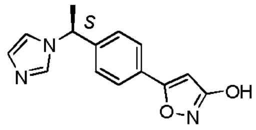 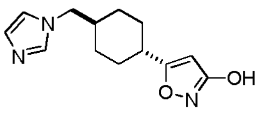 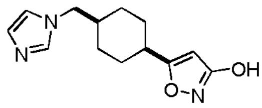 .

Relevant Definitions:

The $C_{1-6}$ is selected from the group consisting of $C_1$, $C_2$, $C_3$, $C_4$, $C_5$ and $C_6$, the $C_{3-6}$ is selected from the group consisting of $C_3$, $C_4$, $C_5$ and $C_6$, the number indicates the number of carbon atoms.

The 5- to 6- membered cyclohydrocarbyl includes saturated or unsaturated ones, also includes aromatic or aliphatic ones. The 5- to 6- membered unsaturated cyclohydrocarbyl or heterocyclyl includes aryl, heteroaryl and aliphatic ones.

An $C_{1-6}$ alkyl, a $C_{1-6}$ heteroalkyl, a $C_{3-6}$ cycloalkyl, a $C_{3-6}$ heterocycloalkyl, an $C_{1-6}$ alkyl substituted by a $C_{3-6}$ cycloalkyl or a $C_{3-6}$ heterocycloalkyl, and a $C_{1-6}$ heteroalkyl substituted by a $C_{3-6}$ cycloalkyl or a $C_{3-6}$ heterocycloalkyl include, but are not limited to, methyl, ethyl, *n*-propyl, isopropyl, $-CH_2C(CH_3)(CH_3)(OH)$, cyclopropyl, cyclobutyl, propyl methyl, cyclopropyl acyl, benzyloxy, cyclopropylenyl, trifluoromethyl, aminomethyl, hydroxymethyl, methoxy, methylacyl, methoxyacyl, methylsulfonyl, methylsulfinyl, ethoxy, acetyl, ethylsulfonyl, ethoxyacyl, dimethylamino, diethylamino, dimethylamino, and diethylamino; $N(CH_3)_2$, $NH(CH_3)$, $-CH_2CF_3$, - $CH_2CH_2CF_3$, $-CH_2CH_2F$, $-CH_2CH_2S(=O)_2CH_3$, $-CH_2CH_2CN$,

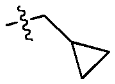 , 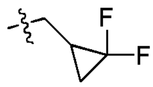 ,

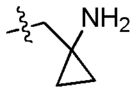 , 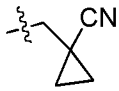 , 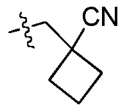 , 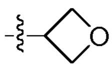 ,

$-CH_2CH(OH)(CH_3)_2$, $-CH_2CH(F)(CH_3)_2$, - $CH_2CH_2F$, $-CH_2CF_3$, $-CH_2CH_2CF_3$, $-CH_2CH_2NH_2$, $-CH_2CH_2OH$, $-CH_2CH_2OCH_3$, - $CH_2CH_2CH_2OCH_3$, $-CH_2CH_2N(CH_3)_2$, $-S(=O)_2CH_3$, $-CH_2CH_2S(=O)_2CH_3$,

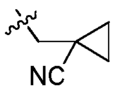 , 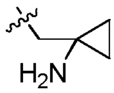 ,

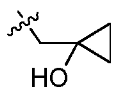 , 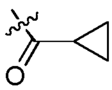 , 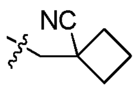 , 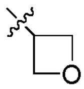 , 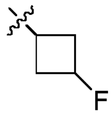 , 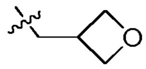 , 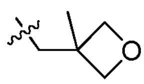 ,

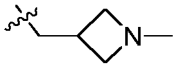 .

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically acceptable salt" is meant to include a salt of a compound of the present invention which is prepared by a relatively nontoxic acid or base with the compound of the present invention having particular substituents. When the compound of the present invention contains a relatively acidic functional group, a base addition salt can be obtained by contacting a neutral form of such compounds with a sufficient amount of a desired base, either neat or in a suitable inert solvent. Examples of the pharmaceutically acceptable base addition salts include salts of

sodium, potassium, calcium, ammonium, organic amine, or magnesium, or similar salts. When the compound of the present invention contains a relatively basic functional group, an acid addition salt can be obtained by contacting a neutral form of such compounds with a sufficient amount of a desired acid, either neat or in a suitable inert solvent. Examples of the pharmaceutically acceptable acid addition salts include salts of inorganic acids including hydrochloric, hydrobromic, nitric, carbonic, hydrocarbonic, phosphoric, hydrophosphoric, dihydrophosphoric, sulfuric, hydrosulfuric, hydriodic, or phosphorous acids and the like; as well as salts of organic acids including acetic, propionic, isobutyric, maleic, malonic, benzoic, succinic, suberic, fumaric, lactic, mandelic, phthalic, benzenesulfonic, p-tolylsulfonic, citric, tartaric, methanesulfonic acid, or the like; and also salts of amino acids (such as arginate and the like), and salts of organic acids like glucuronic acid and the like (see, for example, Berge et al., "Pharmaceutical Salts", Journal of Pharmaceutical Science 66: 1-19 (1977)). Certain specific compounds of the present invention contain both basic and acidic functionalities that allow the compounds to be converted into either base or acid addition salts.

The neutral form of the compound is preferably regenerated by contacting the salt with a base or acid and then isolating the parent compounds in the conventional manner. The parent form of the compound differs from the various salt forms thereof in certain physical properties, such as solubility in polar solvents.

As used herein, the term "pharmaceutically acceptable salts" refers to derivatives of the compound of the present invention wherein the parent compound is modified by making a salt with an acid or base. Examples of pharmaceutically acceptable salts include, but are not limited to, inorganic or organic acid salts of basic groups such as amines; alkali or organic salts of acidic groups such as carboxylic acids and the like. The pharmaceutically acceptable salts include the conventional non-toxic salts or the quaternary ammonium salts of the parent compound formed, for example, from non- toxic inorganic or organic acids. Such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, ethane sulfonic, fumaric, glucoheptonic, gluconic, glutamic, glycolic, hydrobromic, hydrochloric, hydroiodide, hydroxyl acids, hydroxynaphthoic, isethionate, lactic, lactobionic, lauryl sulfonic, maleic, malic, mandelic, methane sulfonic, nitric acid, oxalic acid, pamoic pamoic acid, pantothenic, phenylacetic, phosphoric, polygalacturonic, propionic, salicyclic, stearic, folinate, succinic, sulfamic, sulfanilic, sulfuric acid, tannic, tartaric, and *p*-toluene sulfonic acid.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile or the like are preferred.

In addition to salt forms, the present invention also provides compounds which are in a prodrug form. Prodrugs of the compounds described herein readily undergo chemical changes under physiological conditions to provide the compounds of the present invention. Additionally, prodrugs can be converted to the compound of the present invention by chemical or biochemical methods in an in vivo environment.

Certain compounds of the present invention can exist in unsolvated forms or solvated forms, including hydrated forms. In general, the solvated forms are equivalent to unsolvated forms and all are encompassed within the scope of the present invention.

Certain compounds of the present invention may possess asymmetric carbon atoms (optical centers) or double bonds. The racemates, diastereomers, geometric isomers and individual isomers are all encompassed within the scope of the present invention.

The graphic representations of racemic, ambiscalemic and scalemic or enantiomerically pure compounds used herein are taken from Maehr, J. Chem. Ed. 1985, 62: 114-120. Solid wedge and broken wedge are used to denote the absolute configuration of a stereocenter unless otherwise noted. When the compounds described herein contain olefinic double bonds or other centers of geometric asymmetry, and unless specified otherwise, it is intended that the compounds include both *E* and *Z* geometric isomers. Likewise, all tautomeric forms are included.

Compounds of the present invention can exist in particular geometric or stereoisomeric forms. The invention contemplates all such compounds, including *cis*- and *trans*-isomers, (-)- and (+)-enantiomers, (*R*)- and (*S*)-enantiomers, diastereomers, (*D*)-isomers, (*L*)-isomers, the racemic mixtures thereof, and other mixtures thereof, such as enantiomerically or diastereomerically enriched mixtures, and all these mixtures as falling within the scope of the invention. Additional asymmetric carbon atoms can be present in a substituent such as an alkyl group. All such isomers, as well as mixtures thereof, are intended to be included in this invention.

Optically active (*R*)- and (*S*)-isomers and *D* and *L* isomers can be prepared by chiral synthons or chiral reagents, or other conventional techniques. If, a particular enantiomer of a compound of the present invention is desired, it can be prepared by asymmetric synthesis, or by derivatization with a chiral auxiliary, where the resultant diastereomeric mixture is separated and the auxiliary group is cleaved to provide the pure desired enantiomers. Alternatively, where the molecule contains a basic functional group (such as an amino group) or an acidic functional group (such as a carboxyl group), diastereomeric salts can be formed with an appropriate optically active acid or base, followed by resolution of

the diastereomers by general means known in the art, and subsequent recovery of the pure enantiomers. In addition, separation of enantiomers and diastereomers is frequently accomplished by chromatography employing chiral, stationary phases, optionally in combination with chemical derivatization (e.g., formation of carbamates from amines).

The compounds of the present invention may also contain unnatural proportions of atomic isotopes at one or more of the atoms that constitute such compounds. For example, the compounds may be radiolabeled with radioactive isotopes, such as for example tritium ($^3H$), iodine-125 ($^{125}I$) or carbon-14 ($^{14}C$). All isotopic variations of the compounds of the invention, regardless of radioactivity or not, are intended to be encompassed within the scope of the present invention.

The term "pharmaceutically acceptable carrier" refers to any formulation or carrier medium that is capable of delivery of an effective amount of an active agent of the present invention, and does not interfere with the biological activity of the active agent, without toxic side effects in a host or patient. Representative carriers include water, oils, both vegetable and mineral, cream bases, lotion bases, ointment bases and the like. These bases include suspending agents, thickeners, penetration enhancers, and the like. Their formulation is well known to those in the art of cosmetics and topical pharmaceuticals. Additional information concerning carriers can be found in Remington: The Science and Practice of Pharmacy, 21st Ed., Lippincott, Williams & Wilkins (2005), which is incorporated herein by reference.

The term "excipients" conventionally means carriers, diluents and/or vehicles needed in formulating effective pharmaceutical compositions.

For drugs or pharmacologically active agents, the terms "effective amount" or "therapeutically effective amount" refers to a nontoxic but sufficient amount of the drug or agent to provide the desired effect. In the oral dosage forms of the present disclosure, an "effective amount" of an active angent of the composition refers to the amount of the activeagent required to provide the desired effect when used in combination with the other active agent of the composition. The amount that is "effective" will vary from subject to subject, depending on the age and general condition of a recipient, and also a particular active agent, and an appropriate effective amount in an individual case may be determined by one of ordinary skill in the art using routine experimentation.

The terms "active ingredient," "therapeutic agent," "active aubstance," or "active agent" mean a chemical entity which can be effective in treating a targeted disorder, disease or condition.

The term "substituted", means that any one or more hydrogens on a designated atom is replaced with a substituent including deuterium and a variant of hydrogen, provided that the designated atom’s valency is normal, and that the substituted compound is stable. When a substituent is oxo (i.e., =O), it means that 2 hydrogen atoms are replaced. Oxo substituents are not present on aromatic moieties. The term "optionally substituted" means that the designated atom can be substituted or unsubstituted, and unless otherwise stated, the species and number of the substituents may be arbitrary provided that they can be achieved in chemistry.

When any variable (e.g., R) occurs more than once in the constituent or structure of a compound, its definition at each occurrence is independent. Thus, for example, if a group is substituted with 0-2 R(s), then said group may optionally be substituted with up to two R groups and R at each occurrence has independently options. Also, combinations of substituents and/or variables are permissible only if such combinations result in stable compounds.

When a substituent is shown to cross a bond connecting two atoms in a ring, then such substituent may be bonded to any atom on the ring. When a substituent is listed without indicating the atom via which such substituent is bonded to the rest of the compound, then such substituent may be bonded via any atom therein. Combinations of substituents and/or variables are permissible only if such combinations result in stable compounds.

Substituents for the alkyl and heteroalkyl radicals (including the groups commonly named as alkylene, alkenyl, heteroalkylene, heteroalkenyl, alkynyl, cycloalkyl, heterocycloalkyl, cycloalkenyl and heterocycloalkenyl) are generically referred to as "alkyl group substituents" and they can be one or more of a variety of groups selected from, but not limited to: -R’, -OR’, =O, =NR’, =N-OR’, -NR’R", -SR’, -halogen, -SiR’R"R’’’, OC(O)R’, -C(O)R’, $-CO_2R'$, -CONR’R", -OC(O)NR’R", -NR"C(O)R’, NR’ C(O)NR"R’’’, $-NR''C(O)_2R'$, -NR’’’’-C(NR’R"R’’’)=NR’’’’, NR’’’’ C(NR’R")=NR’’’, -S(O)R’, $-S(O)_2R'$, $-S(O)_2NR'R''$, $NR''SO_2R'$, -CN, $-NO_2$, $-N_3$, $-CH(Ph)_2$, and fluoro($C_1$-$C_4$)alkyl, in a number ranging from zero to (2m’+1), where m’ is the total number of carbon atoms in such radical. Preferably each of R’, R", R’’’, R’’’’ and R’’’’’ is independently selected from hydrogen, substituted or unsubstituted heteroalkyl, substituted or unsubstituted aryl(e.g., an aryl substituted with 1 to 3 of halogens), substituted or unsubstituted alkyl, alkoxy or thioalkoxy groups, or arylalkyl groups. When a compound of the present invention includes more than one R group, for example, each of the R groups is independently selected as are each R’, R", R’’’, R’’’’ and R’’’’’ groups when more than one of these groups is present. When R’ and R" are attached to the same nitrogen atom, they can be combined with the nitrogen atom to form a 5-, 6-, or 7-membered ring. For example, -NR’R" is meant to include, but not be limited to, 1-pyrrolidinyl and 4-morpholinyl. From the above discussion of substituents, one person skilled in the art will understand that the term "alkyl" is meant to include groups including carbon atoms bound to groups other than hydrogen groups, such as haloalkyl (e.g., $-CF_3$ and $-CH_2CF_3$) and acyl (e.g., $-C(O)CH_3$, $-C(O)CF_3$, $-C(O)CH_2OCH_3$, and the like). Similar to the substituents described for the alkyl radical, substituents for the aryl and heteroaryl groups are generically referred to as "aryl group substituents." The substituents are selected from, for example: -R’, -OR’, -NR’R", - SR’, -halogen, -SiR’R"R’’’, OC(O)R’, -C(O)R’, $-CO_2R'$,

-CONR'R", -OC(O)NR'R", - NR"C(O)R', NR' C(O)NR"R"', -NR"C(O)$_2$R', -NR""'-C(NR'R"R"')=NR"", NR"" C(NR'R")=NR"', -S(O)R', -S(O)$_2$R', -S(O)$_2$NR'R", NR"SO$_2$R', -CN, -NO$_2$, -N$_3$, -CH(Ph)$_2$, fluoro($C_1$-$C_4$)alkoxy, and fluoro($C_1$-$C_4$)alkyl, in a number ranging from zero to the total number of open valences on the aromatic ring system; and wherein each of R', R", R"', R"" and R""' is preferably and independently selected from hydrogen, substituted or unsubstituted alkyl, substituted or unsubstituted heteroalkyl, substituted or unsubstituted aryl and substituted or unsubstituted heteroaryl. When a compound of the present invention includes more than one R group, for example, each of the R groups is independently selected as are each R', R", R"', R"" and R""' groups when more than one of these groups is present.

The two substituents on adjacent atoms of an aryl or heteroaryl ring may be optionally substituted by substituents of the general formula -TC(O)-(CRR')q-U-, wherein each of T and U is independently selected from -NR -, -O-, CRR'- and a single bond, and q is an integer from 0 to 3. Alternatively, the two substituents on adjacent atoms of an aryl or heteroaryl ring may be optionally substituted by substituents of the general formula -A ($CH_2$) rB-, wherein each of A and B is independently selected from -CRR'-, -O-, -NR-, -S-, -S(O)-, S(O)$_2$-, -S(O)$_2$NR'-or a single bond, r is an integer from 1 to 4. Optionally, a single bond on the resulting new ring may be replaced by a double bond. Alternatively, the two substituents on adjacent atoms of an aryl or heteroaryl ring may be optionally substituted by substituents of the general formula -A ($CH_2$) rB-, wherein, each of s and d is independently selected from an integer from 0 to 3, and X is -O-, -NR', -S-, -S(O)-, - S(O)$_2$-or -S(O)$_2$NR'-. Each substituent R, R', R" or R"' is independently selected from hydrogen and substituted or unsubstituted ($C_1$-$C_6$) alkyl.

The terms "halo" or "halogen" by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo($C_1$-$C_4$)alkyl" is mean to include, but not be limited to, trifluoromethyl, 2,2,2-trifluoroethyl, 4-chlorobutyl, 3-bromopropyl, and the like.

Examples of haloalkyl include, but are not limited to, trifluoromethyl, trichloromethyl, pentafluoroethyl, and pentachloroethyl. "Alkoxy" represents an alkyl group as defined above with the indicated number of carbon atoms attached through an oxygen bridge. $C_{1-6}$ alkoxy, is intended to include $C_1$, $C_2$, $C_3$, $C_4$, $C_5$, and $C_6$ alkoxy groups. Examples of alkoxy include, but are not limited to, methoxy, ethoxy, *n*-propoxy, *i*-propoxy, *n*-butoxy, *s*-butoxy, *t*-butoxy, *n*-pentoxy, and *s*-pentoxy. "Cycloalkyl" is intended to include saturated ring groups, such as cyclopropyl, cyclobutyl, or cyclopentyl. 3-7 cycloalkyl is intended to include $C_3$, $C_4$, $C_5$, $C_6$, and $C_7$ cycloalkyl groups. "Alkenyl" is intended to include hydrocarbon chains of either linear or branched configuration and one or more carbon-carbon double bonds that may occur in any stable site along the chain, such as ethenyl and propenyl.

The term "halo" or "halogen" as used herein refers to fluoro, chloro, bromo, and iodo.

As used herein, the term "hetero", "heteroatom" or "heteroatomic radical" (namely radical containing heteroatom), unless otherwise stated, include atoms other than carbon (C) and hydrogen (H), also include the radicals containing these aforesaid heteroatoms. Examples include oxygen (O), nitrogen (N) sulfur (S), silicon (Si), germanium (Ge), aluminum (Al) and boron (B) etc., also include optionally substituted -C(=O)N(H)-, -N(H)-, -C(=NH)-, -S(=O)$_2$ N(H)-, or -S(=O) N(H)-.

"Ring or cyclo" means a substituted or unsubstituted cycloalkyl, a substituted or unsubstituted heterocycloalkyl, a substituted or unsubstituted aryl, or a substituted or unsubstituted heteroaryl. The so-called ring includes fused ring. The number of atoms in a ring is typically defined as the number of members of the ring. For example, a "5- to 7-membered ring" means there are 5 to 7 atoms in the encircling arrangement. Unless otherwise specified, the ring optionally includes one to three heteroatoms. Thus, the term "5- to 7-membered ring" includes, for example phenyl, pyridinyl and piperidinyl. The term "5- to 7-membered heterocycloalkyl ring", on the other hand, include pyridinyl and piperidinyl, but not phenyl. The term "ring" further includes a ring system comprising at least one ring, wherein each "ring" is independently defined as above.

The term "heterocycle" or "heterocyclo-" is intended to mean a stable monocyclic, or a bicyclic, or a heterobicyclic, which may be saturated, partially unsaturated or unsaturated (aromatic), and include carbon atoms and 1, 2, 3, or 4 of ring heteroatoms independently selected from the group consisting of N, O and S in which any of the above-defined heterocyclic rings may be fused to a benzene ring to form a bicyclic group. The nitrogen and sulfur heteroatoms may optionally be oxidized (i. e., NO and S (O) p). The nitrogen atom may be substituted or unsubstituted (i.e., N or NR, wherein R is H or other substituents, already defined herein). The heterocyclic ring may be attached to its side group at any heteroatom or carbon atom that results in a stable structure. The heterocyclic rings described herein may be substituted on carbon or on a nitrogen atom if the resultant compound is stable. A nitrogen in the heterocycle may optionally be quaternized. In a preferred embodiment, when the total number of S and O atoms in the heterocycle exceeds 1, then these heteroatoms are not adjacent to one another. In another preferred embodiment the total number of S and O atoms in the heterocycle is not more than 1. As used herein, the term "aromatic heterocyclic group" or "heteroaryl" is intended to mean a stable 5-, 6-, or 7-membered monocyclic or bicyclic or 7-, 8-, 9-, or 10-membered bicyclic heterocyclic aromatic ring which includes carbon atoms and 1, 2, 3, or 4 of heterotams independently selected from the group consisting of N, O and S. The nitrogen atom may be substituted or unsubstituted (i.e., N or NR, wherein R is H or other substituents

already defined herein). The nitrogen and sulfur heteroatoms may optionally be oxidized (i.e., NO and S (O) p). It is to be noted that total number of S and O atoms in the aromatic heterocycle is not more than 1. Bridged rings are also included in the definition of heterocycle. A bridged ring occurs when one or more atoms (i.e., C, O, N, or S) link two non-adjacent carbon or nitrogen atoms. Preferred bridged rings include, but are not limited to, one carbon atom, two carbon atoms, one nitrogen atom, two nitrogen atoms, and a carbon-nitrogen group. It is to be noted that a bridge always converts a monocyclic ring into a tricyclic ring. In a bridged ring, the substituents on the ring may also be present on the bridge.

Examples of heterocycles include, but are not limited to, acridinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, dihydrofuro [2,3-b] tetrahydrofuran, furanyl, furazanyl, imidazolidinyl, imidazolinyl, imidazolyl, 1H-indazolyl, isatinoyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2, 4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds.

The term "hydrocarbyl" or its hyponyms (such as alkyl, alkenyl, alkynyl and phenyl etc.) by itself or as part of another substituent, means, unless otherwise stated, a linear or branched chain, or cyclic hydrocarbon radical, or combination thereof, which may be fully saturated, mono- or polyunsaturated, may be mono- or polysubstituted, may be monovalent (e.g., methyl), divalent (e.g., methylene), or polyvalent (e.g., methyne), and can include di- or multivalent radicals, having the designated number of carbon atoms designated (i.e. $C_1$-$C_{10}$ meaning 1 to 10 carbons). "Hydrocarbyl" include, but are not limited to, aliphatic hydrocarbyl and aromatic hydrocarbyl, and the aliphatic hydrocarbyl include linear and cyclic ones, specifically including but not limited to, alkyl, alkenyl, and alkynyl, and the aromatic hydrocarbyl includes, but are not limited to, 6- to 12-membered aromatic hydrocarbyl, for example, benzene, and naphthalene, etc. In some embodiments, the term "hydrocarbyl" means a linear or branched chain radical, or combinations thereof, which may be fully saturated, mono- or polyunsaturated and can include di- and multivalent radicals. Examples of saturated hydrocarbon radicals include, but are not limited to, methyl, ethyl, *n*-propyl, isopropyl, *n*-butyl, *t*-butyl, isobutyl, sec-butyl, cyclohexyl, (cyclohexyl)methyl, cyclopropylmethyl, homologs and isomers of radicals such as *n*-pentyl, *n*-hexyl, *n*-heptyl, *n*-octyl, and the like. An unsaturated alkyl group is one having one or more double bonds or triple bonds. Examples of unsaturated hydrocarbyl groups include, but are not limited to, vinyl, 2-propenyl, butenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers.

The term "heterohydrocarbyl" or its hyponyms (such as heteroalkyl, heteroalkenyl, heteroalkynyl and heteroaryl etc.) by itself or in combination with another term, means, unless otherwise stated, a stable linear or branched chain, or cyclic hydrocarbon radical, or combinations thereof, consisting of the stated number of carbon atoms and at least one heteroatom. In some embodiments, the term "heteroalkyl" by itself or in combination with another term, means a stable linear or branched chain alkyl radical, or combinations thereof, consisting of the stated number of carbon atoms and at least one heteroatom. In a typical embodiment, the heteroatoms are selected from the group consisting of B, O, N and S, wherein the nitrogen and sulfur atoms may optionally be oxidized and the nitrogen heteroatom may optionally be quaternized. The heteroatoms B, O, N and S may be placed at any internal position of the heterohydrocarbyl group, (including the position at which the hydrocarbyl group is attached to the remainder of the molecule). Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2,-S(O)-CH_3$, $-CH_2-CH_2-S(O)_2-CH_3$, $-CH=CH-O-CH_3$, $-CH_2-CH=N-OCH_3$, and $-CH=CH-N(CH_3)-CH_3$. Up to two heteroatoms may be consecutive, such as, for example, $-CH_2-NH-OCH_3$.

The terms "alkoxy," "alkylamino" and "alkylthio" (or thioalkoxy) are used in their conventional sense, and refer to those alkyl groups attached to the remainder of the molecule via an oxygen atom, an amino group, or a sulfur atom, respectively.

The terms "cyclohydrocarbyl", "heterocyclohydrocarbyl", "cycloheterohydrocarbyl" or their hyponyms (such as aryl, heteroaryl, aromatic heterohydrocarbyl, cycloalkyl, heterocycloalkyl, cycloalkyl heterohydrocarbyl, cycloalkenyl, heterocycloalkenyl, cycloalkenyl heterohydrocarbyl, cycloalkynyl, heterocycloalkynyl, cycloalkynyl heterohydrocarbyl, etc.) by themselves or in combination with other terms, represent, unless otherwise stated, cyclic versions of "hydrocarbyl" "heterohydrocarbyl", hydrocarbyl heterohydrocarbyl, respectively. Additionally, for heterohydrocarbyl or heterocyclohydrocarbyl (such as heteroalkyl and heterocycloalkyl), a heteroatom can occupy the position at which the heterocycle is

attached to the remainder of the molecule. Examples of cycloalkyl include, but are not limited to, cyclopentyl, cyclohexyl, 1-cyclohexenyl, 3-cyclohexenyl, cycloheptyl, and the like. Non-limiting examples of heterocycle moieties include 1-(1,2,5,6-tetrahydropyridyl), 1-piperidinyl, 2-piperidinyl, 3-piperidinyl, 4-morpholinyl, 3-morpholinyl, tetrahydrofuranindol-2-yl, tetrahydrofuran-3-yl, tetrahydrothien-2-yl, tetrahydrothien-3-yl, 1 -piperazinyl, and 2-piperazinyl.

The term "aryl" means, unless otherwise stated, a polyunsaturated, aromatic substituent that may be mono-, di- or poly-substituted, and can be monovalent, divalent, or polyvalent, or a single ring or multiple rings (preferrably 1 to 3 rings), which are fused together or linked covalently. The term "heteroaryl" refers to aryl groups (or rings) that contain from one to four heteroatoms. In an exemplary embodiment, the heteroatom is selected from the group consisting of B, N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents of any of the above described aryl and heteroaryl ring systems are selected from the acceptable substituents described below.

For brevity, the term "aryl" when used in combination with other terms (e.g., aryloxy, arylthio, arylalkyl) includes both aryl and heteroaryl rings as defined above. Thus, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom, e.g., phenoxymethyl, 2-pyridyloxymethyl, 3-(1-naphthyloxy)propyl, and the like.

The term "leaving group" means a functional group or atom which can be displaced by another functional group or atom in a substitution reaction, (such as a nucleophilic substitution reaction). For example, representative leaving groups include triflate, chloro, bromo and iodo groups; sulfonic ester groups, such as mesylate, tosylate, brosylate, nosylate and the like; and acyloxy groups, such as acetoxy, trifluoroacetoxy and the like.

The term "protecting group" includes but is not limited to "amino-protecting group", "hydroxy-protecting group" or "thiol-protecting group". The term "amino-protecting group" means a protecting group suitable for preventing side reactions at an amino nitrogen. Representative amino-protecting groups include, but are not limited to, formyl; acyl groups, for example alkanoyl groups, such as acetyl, trichloroacetyl or trifluoroacetyl; alkoxycarbonyl groups, such as tert-butoxycarbonyl (Boc); arylmethoxycarbonyl groups, such as benzyloxycarbonyl (Cbz) and 9-fluorenylmethoxycarbonyl (Fmoc); arylmethyl groups, such as benzyl (Bn), trityl (Tr), and 1,1-di-(4'-methoxyphenyl)methyl; silyl groups, such as trimethylsilyl (TMS) and tert-butyldimethylsilyl (TBS); and the like. The term "hydroxy-protecting group" means a protecting group suitable for preventing side reactions at a hydroxy group. Representative hydroxy-protecting groups include, but are not limited to, alkyl groups, such as methyl, ethyl, and tert-butyl; acyl groups, for example alkanoyl groups, such as acetyl; arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The present invention is now further described by way of examples. The examples given below are for illustrative purposes only and are not intended to be limited to the scope of the invention. The compounds of the present invention can be prepared by a number of known methods in the field of organic synthesis. Embodiments of the present invention can be synthesized using the methods described below, as well as synthetic methods known in the art of organic synthetic chemistry, or on the basis of which are improved. Preferred methods include, but are not limited to, the methods described below.

All of the solvents used in the present invention are commercially available and can be used without further purification. The reaction is generally carried out under inert nitrogen and in an anhydrous solvent. Proton nuclear magnetic resonance data is recorded on the spectrometer of Bruker Avance III 400 (400 MHz), and a chemical shift is represented by (ppm) at the low field of tetramethylsilane. The mass spectrum is measured on the Agilent 1200 Series Plus 6110 (& 1956A). LC/ MS or Shimadzu MS contains a DAD: SPD-M20A(LC) and Shimadzu Micromass 2020 detector. The mass spectrometer is equipped with an electrospray ion source (ESI) operating in positive or negative mode.

The following abbreviations are used: aq represents aqueous; DCM represents dichloromethane; PE represents petroleum ether; DMF represents *N*,*N*-dimethylfornamide; DMSO represents dimethylsulfoxide; EtOAc represents ethyl acetate; EtOH represents ethanol; MeOH represents methanol; CBz represents benzyloxycarbonyl, a amine protecting group; BOC represents *tert*-butylcarbonyl, amine protecting group; HOAc represents acetic acid; $NaBH(OAc)_3$ represents sodium triacetoxyborohydride; r.t. represents room temperature; THF represents tetrahydrofuran; $Boc_2O$ represents di-*tert*-butyl dicarbonate; TFA represents trifluoroacetic acid; DIPEA represents diisopropylethylamine; $Pd(dppf)Cl_2$ represents [1,1'-bis (diphenylphosphino) ferrocene] palladium (II) dichloride; $POCl_3$ represents phosphorus oxychloride; NaH represents sodium hydride; LAH represents lithium aluminum hydride; $Pd(OAc)_2$ represents palladium (II) acetate; $Pd_2(dba)_3$ represents tris(dibenzylideneacetone)dipalladium; $Pd(PPh_3)_4$ represents tetrakis(triphenylphosphine)palladium; $Et_3SiH$ represents triethylsilane; $PPh_3$ represents triphenylphosphine; Xantphos represents 4,5-bis(diphenylphos-

phino) -9,9-dimethyl; $MeSO_3H$ represents methanesulfonic acid; Xphos represents 2-dicyclohexylphosphino-2',4',6'-triisopropylbiphenyl; Lawesson reagent represents 2,4-bis (4-methoxyphenyl)-1,3-dithia-2,4-diphosphane-2,4-disulfide; NBS represents N-Bromosuccinimide; t-BuOK represents potassium tert-butoxide.

Compounds were named either manually or by using ChemDraw®, or using vendors catalogue name if commercially available.

DETAILED DESCRIPTION OF THE PREFERRED EMBODIMENT

For the purpose of illustrating the invention in greater detail, the following examples are given, but the scope of the invention is not limitedthereto.

**Scheme A**

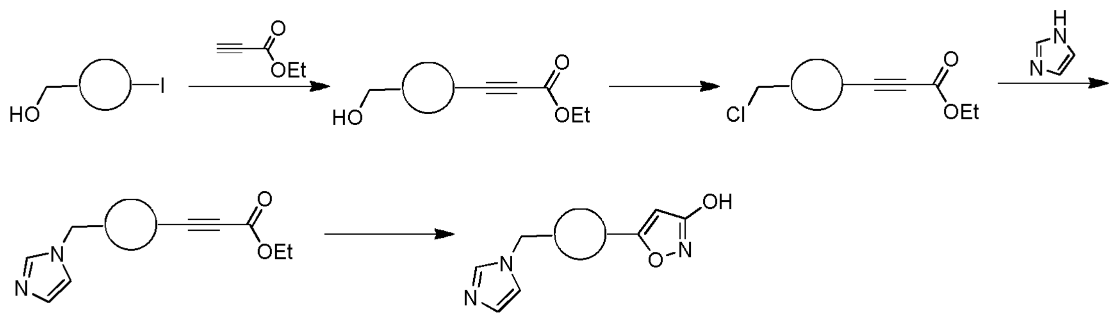

**Example 1**

**5-(4-((1*H*-imidazol-1-yl)methyl)phenyl)isoxazol-3-ol**

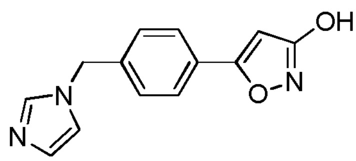

**Compound 1A**

**Ethyl 3-(4-(hydroxymethyl)phenyl)propiolate**

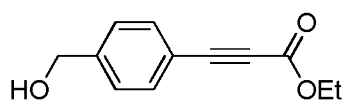

4-Iodobenzoyl alcohol (73 g, 311 mmol), ethyl propiolate (91.4 g, 933 mmol) and $Cu_2O$ (44.6 g, 311 mmol) was added to DMF (700 mL) under $N_2$ atmosphere. The solution was stirred at 110°C for 8 hours, cooled to room temperature and filtered. The filtrate was concentrated under reduced pressure to remove most of DMF. To the residue was added water (400 mL). The mixture was extracted with EtOAc (300 mL*3). The combined organic layers were dried over anhydrous $Na_2SO_4$, filtered and concentrated to give the crude product. The crude product was purified by column chromatography to give **Compound 1A** (light-gray solid, 45 g, 70% yield). LCMS (ESI) m/z: 205($M+H^+$).

**Compound 1B**

**Ethyl 3-(4-(chloromethyl)phenyl)propiolate**

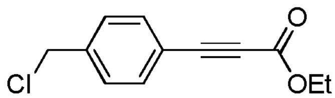

To a solution of **Compound 1A** (45 g, 225 mmol) and DMF (0.5 mL) in DCM (200 mL) was added dropwise $SOCl_2$ (104 g, 881 mmol) at 0°C. After the addition, the mixture was stirred at 20°C for 1 hour and concentrated under reduced pressure to remove most of solvent and $SOCl_2$. The residue was purified by column chromatography to give **Compound 1B** (colorless liquid 37.5 g, 76.4% yield). $^1H$ NMR (400 MHz, $CDCl_3$): δ 7.58 (d, *J* = 8.4 Hz, 2 H), 7.41 (d, *J* = 8.4 Hz, 2 H), 4.59 (s, 2 H), 4.31 (q, *J* = 7.2 Hz, 2 H), 1.36 (t, *J* = 7.2 Hz, 3 H). LCMS (ESI) m/z: 223(M+$H^+$).

**Compound 1C**

**Ethyl 3-(4-((1*H*-imidazol-1-yl)methyl)phenyl)propiolate**

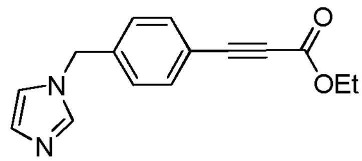

**Compound 1B** (37 g, 161 mmol), imidazole (22 g, 322 mmol), KI (22 g, 322 mmol) and $K_2CO_3$ (44.7 g, 323 mmol) was added into acetone (370 mL). The solution was heated to 50-60°C, stirred for 3 hours, cooled to room temperature and filtered. The filtrate was poured into water (1200 mL). The mixture was extracted with EtOAc (600 mL*3). The combined organic layers were dried over anhydrous $Na_2SO_4$, filtered and concentrated under reduced pressure. The residue was purified by column chromatography to give **Compound 1C** (white solid, 18 g, 32.2% yield). $^1H$ NMR (400 MHz, $CDCl_3$): δ7.50-7.63 (m, 3 H), 7.07-7.18 (m, 3 H), 6.90 (s, 1 H), 5.16 (s, 2 H), 4.30 (q, *J* = 7.2 Hz, 2 H), 1.35 (t, *J* = 7.2 Hz, 3 H). LCMS (ESI) m/z: 255(M+$H^+$).

**Compound 1**

**5-(4-((1*H*-imidazol-1-yl)methyl)phenyl)isoxazol-3-ol**

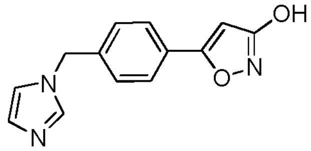

Solid NaOH (14.2 g, 350 mmol) was dissolved into water (60 mL) at 0°C. To this solution was added $NH_2OH \cdot HCl$ (14.8 g, 350 mmol) in batches, after being stirred for 10 mins, this solution was added to a solution of **Compound 1C** (18 g, 70.8 mmol) in MeOH (60 mL) in batches. The mixture was heated to 30-40°C, stirred for 3 hours and concentrated under reduced pressure to remove most of solvent. The residue was acidized by 4M HCl-MeOH solution (200 mL), filtered and concentrated ,the crude product was triturated in EtOH (100 mL) for 1 hour and filtered to give the hydrochloride salt of **Compound 1** (6.5 g, 36% yield). $^1H$ NMR (400 MHz, $CD_3OD$): δ 9.12 (s, 1 H), 7.86 (d, *J* = 8.28 Hz, 2 H), 7.69 (s, 1 H), 7.63 (s, 1 H), 7.55 (d, *J* = 8.28 Hz, 2 H), 6.41 (s, 1 H), 5.55 (s, 2 H). LCMS (ESI) m/z: 242(M+$H^+$).

**Example 2**

**5-(4-((1*H*-imidazol-1-yl)methyl)-3-fluorophenyl)isoxazol-3-ol**

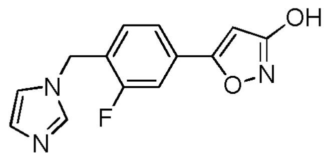

**Compound 2**

Synthesized by the same way as **Example 1.** $^1H$ NMR (400 MHz, $CD_3OD$): δ 9.06 (s, 1 H), 7.49-7.73 (m, 5 H), 6.45 (s, 1 H), 5.57 (s, 2 H). LCMS (ESI) m/z: 260 ($M+H^+$).

**Example 3**

**5-(4-((1*H*-imidazol-1-yl)methyl)-2-fluorophenyl)isoxazol-3-ol**

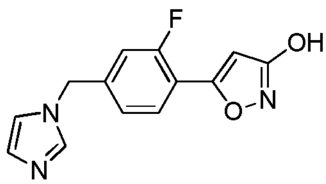

**Compound 3**

Synthesized by the same way as **Example 1.** $^1H$ NMR (400 MHz, $CD_3OD$): δ 9.14 (s, 1 H), 7.97 (t, *J* = 7.78 Hz, 1 H), 7.72 (s, 1 H), 7.65 (s, 1 H), 7.17-7.49 (m, 2 H), 6.38 (d, *J* = 3.51 Hz, 1 H), 5.57 (s, 2 H). LCMS (ESI) m/z: 260 ($M+H^+$).

**Example 4**

**5-(4-((1*H*-imidazol-1-yl)methyl)-2,5-difluorophenyl)isoxazol-3-ol**

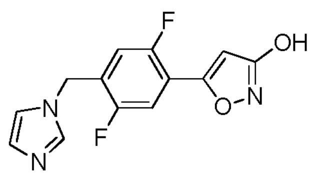

**Compound 4**

Synthesized by the same way as **Example 1.** $^1H$ NMR (400 MHz, METHANOL-$d_4$): δ 8.86 (s, 1H), 7.78 (dd, *J* = 5.73, 9.92 Hz, 1H), 7.62 (s, 1H), 7.55 (s, 1H), 7.50 (dd, *J* = 5.84, 10.25 Hz, 1H), 6.54 (d, *J* = 3.53 Hz, 1H), 5.62 (s, 2H). LCMS (ESI) m/z: 278 ($M+H^+$).

**Example 5**

**5-(4-((1*H*-imidazol-1-yl)methyl)-2,6-difluorophenyl)isoxazol-3-ol**

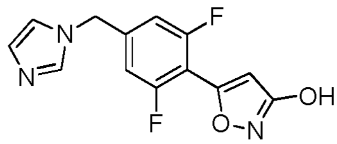

**Compound 5**

Synthesized by the same way as **Example 1.** $^1$H NMR (400MHz, $CD_3OD$): δ9.15 (s, 1H), 7.74 (s, 1H), 7.67 (s, 1H), 7.26 (d, *J* = 9.0 Hz, 2H), 6.39 (s, 1H), 5.57 (s, 2H). LCMS (ESI) m/z: 278 ($M+H^+$).

**Example 6**

**5-(4-((1*H*-imidazol-1-yl)methyl)-3-chloro-5-fluorophenyl)-isoxazol-3-ol**

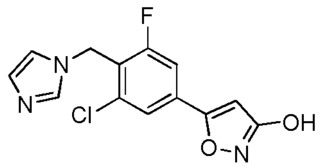

**Compound 6**

Synthesized by the same way as **Example 1.** $^1$H NMR (400MHz, $CD_3OD$): δ 9.07 (s, 1H), 7.87 (s, 1H), 7.73 (d, *J* = 10.0 Hz, 1H), 7.67-7.54 (m, 2H), 6.58 (s, 1H), 5.71 (s, 2H). LCMS (ESI) m/z: 294 ($M+H^+$).

**Example 7**

**5-(4-((1*H*-imidazol-1-yl)methyl)-2-chlorophenyl)isoxazol-3-ol**

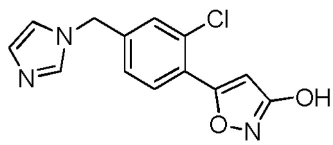

**Compound 7**

Synthesized by the same way as **Example 1.** $^1$H NMR (400MHz, METHANOL-$d_4$): δ 9.15 (s, 1H), 7.96 (d, *J* = 8.2 Hz, 1H), 7.73 (s, 1H), 7.69 (s, 1H), 7.65 (s, 1H), 7.50 (dd, *J* = 1.6, 8.0 Hz, 1H), 6.62 (s, 1H), 5.56 (s, 2H). LCMS (ESI) m/z: 276 ($M+H^+$).

**Example 8**

**5-(4-((1*H*-imidazol-1-yl)methyl)-2-methylphenyl)isoxazol-3-ol**

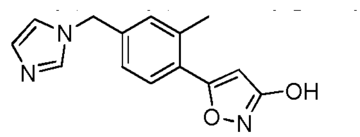

**Compound 8**

Synthesized by the same way as **Example 1.** $^1$H NMR (400MHz, CHLOROFORM-d): δ 9.12 (br. s., 1H), 7.77-7.56 (m, 3H), 7.46-7.30 (m, 2H), 6.22 (s, 1H), 5.50 (s, 2H), 2.49 (br. s., 3H). LCMS (ESI) m/z: 256 ($M+H^+$).

**Example 9**

**5-(4-((1H-imidazol-1-yl)methyl)-2-methoxyphenyl)isoxazol-3-ol**

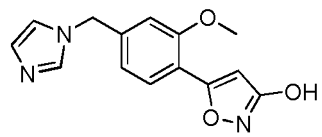

**Compound 9**

Synthesized by the same way as **Example 1.** $^1$H NMR (400MHz, METHANOL-$d_4$): δ 9.16 (s, 1H), 7.87 (d, *J* = 8.0 Hz, 1H), 7.78-7.58 (m, 2H), 7.31 (s, 1H), 7.11 (d, *J* = 8.0 Hz, 1H), 6.44 (s, 1H), 5.54 (s, 2H), 4.02 (s, 3H). LCMS (ESI) m/z: 272 ($M+H^+$).

**Example 10**

**5-(4-((1*H*-imidazol-1-yl)methyl)-3-methoxyphenyl)isoxazol-3-ol**

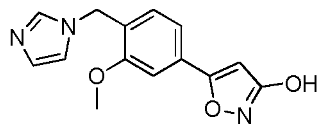

**Compound 10**

Synthesized by the same way as **Example 1.** $^1$H NMR (400MHz, $CD_3OD$): δ 8.73 (s, 1H), 7.39-7.35 (m, 3H), 7.33-7.29 (m, 1H), 7.28-7.24 (m, 1H), 6.06 (s, 1H), 5.37 (s, 2H). LCMS (ESI) m/z: 272 ($M+H^+$).

**Example 11**

**5-(4-((1*H*-imidazol-1-yl)methyl)-3-chlorophenyl)isoxazol-3-ol**

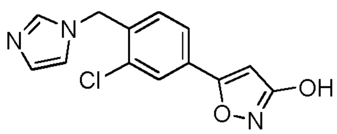

**Compound 11**

Synthesized by the same way as **Example 1.** $^1$H NMR (400MHz, $D_2O$): δ 8.64 (s, 1H), 7.31 (d, *J* = 17.1 Hz, 2H), 7.24 (d, *J* = 7.8 Hz, 1H), 7.03 (d, *J* = 7.8 Hz, 1H), 6.95 (s, 1H), 6.08 (s, 1H), 5.20 (s, 2H). LCMS (ESI) m/z: 276 (M+H$^+$).

**Example 12**

**5-(4-((1*H*-imidazol-1-yl)methyl)-3-methylphenyl)isoxazol-3-ol**

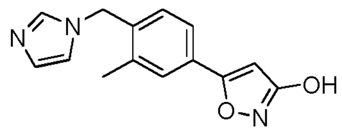

**Compound 12**

Synthesized by the same way as **Example 1.** $^1$H NMR (400MHz, $D_2O$): δ 8.59 (s, 1H), 7.37 (s, 1H), 7.27 (s, 1H), 7.22-7.10 (m, 2H), 7.01 (d, *J* = 7.8 Hz, 1H), 5.94 (s, 1H), 5.26 (s, 2H), 2.04 (s, 3H). LCMS (ESI) m/z: 256 (M+H$^+$).

**Example 13**

**5-(4-((1*H*-imidazol-1-yl)methyl)-3-bromophenyl)isoxazol-3-ol**

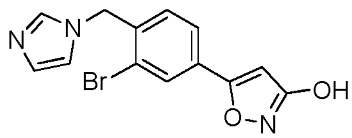

**Compound 13**

Synthesized by the same way as **Example 1.** $^1$H NMR (400MHz, $D_2O$): δ 8.99 (s, 1H), 8.14 (s, 1H), 7.88 (d, *J* = 8.0 Hz, 1H), 7.61 (d, *J* = 13.1 Hz, 2H), 7.53 (d, *J* = 8.0 Hz, 1H), 6.50 (s, 1H), 5.63 (s, 2H). LCMS (ESI) m/z: 321 (M+H$^+$).

**Example 14**

**5-(6-((1*H*-imidazol-1-yl)methyl)pyridin-3-yl)isoxazol-3-ol**

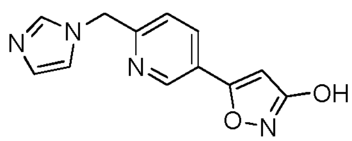

**Compound** 14

Synthesized by the same way as **Example 1.** $^1$H NMR (400 MHz, METHANOL-$d_4$): δ 9.13 (s, 1H), 8.93 (d, *J* = 1.76 Hz, 1H), 8.22 (dd, *J* = 2.21, 8.16 Hz, 1H), 7.70 (s, 1H), 7.57-7.65 (m, 2H), 6.51 (s, 1H), 5.65 (s, 2H). LCMS (ESI) m/z: 243 (M+H$^+$).

**Scheme B**

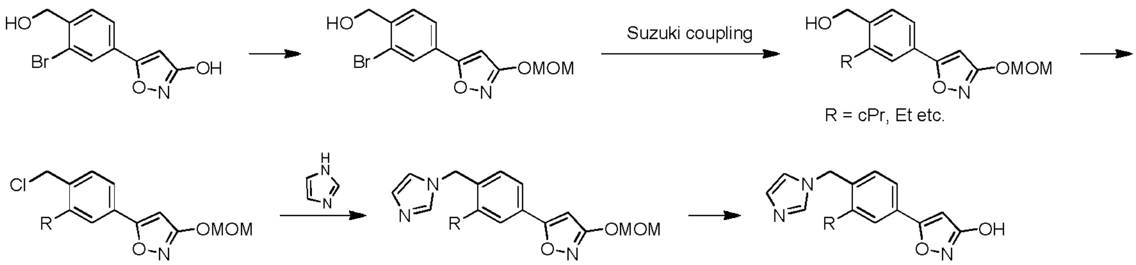


**Example 15**

**5-(4-((1*H*-imidazol-1-yl)methyl)-3-cyclopropylphenyl)isoxazol-3-ol**

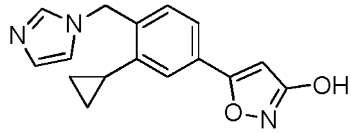

**Compound 15A**

**(2-bromo-4-(3-(methoxymethoxy)isoxazol-5-yl)phenyl)methanol**

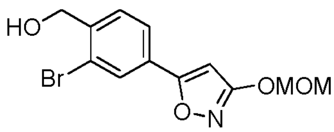

To a solution of 5-(4-((1*H*-imidazol-1-yl) methyl)-3-bromophenyl)isoxazol-3-ol (14.8 g, 350 mmol) and triethylamine (0.8 mL, 5.54 mmol) in DMSO (8 mL) was added dropwise MOMCl (313 mg, 3.89 mmol)at0°C under $N_2$ atmosphere. The mixture was stirred at 0°C for 1 hour, quenched with water (40 mL) and extracted with EtOAc (40 mL*3). The combined organic layers were dried over anhydrous $Na_2SO_4$, filtered and concentrated, the residue was purified by prep-HPLC to give **Compound 15A** (yellow oil, 520 mg, 63.9% yield). LCMS (ESI) m/z: 314 ($M+H^+$).

**Compound 15B**

**(2-cyclopropyl-4-(3-(methoxymethoxy)isoxazol-5-yl)phenyl)methanol**

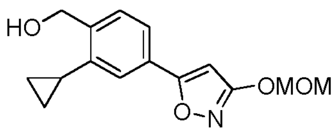

A solution of **Compound 15A** (230 mg, 0.73 mmol), 2-cyclopropyl- 4,4,5,5-tetramethyl-1,3,2-dioxaborolane (369 mg, 2.20 mmol), $K_2CO_3$ (201 mg, 1.46 mmol) and Pd(dppf)$Cl_2$ (54 mg, 0.074 mmol) in 1,4-dioxane (3 mL) and water (0.5 mL) was stirred at 90°C under $N_2$ atmosphere for 2 hour. The mixture was cooled, diluted with water (10 mL) and extracted with EtOAc (15 mL*3). The combined organic layers were dried over anhydrous $Na_2SO_4$, filtered and concentrated, the residue was purified by prep-TLC to give **Compound 15B** (yellow oil, 170 mg, 84.3% yield). LCMS

(ESI) m/z: 276 (M+H+).

**Compound 15C**

**5-(4-(chloromethyl)-3-cyclopropylphenyl)-3-(methoxymethoxy)isoxazole**

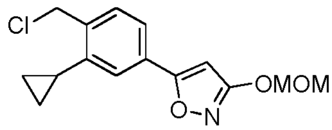

To a solution of **Compound 15B** (170 mg, 0.62 mmol) and TosCl (177 mg, 0.93 mmol) in DCM (5 mL) was added dropwise triethylamine (125 mg, 1.24 mmol) at 0°C under $N_2$ atmosphere. The mixture was stirred at 20°C for 2 hours, diluted with water (10 mL) and extracted with EtOAc (15 mL*3). The combined organic layers were dried over anhydrous $Na_2SO_4$, filtered and concentrated. The residue was purified by prep-TLC to give **Compound 15C** (yellow solid, 45 mg, 17% yield). LCMS (ESI) m/z: 294 (M+H+).

**Compound 15D**

**5-(4-((1*H*-imidazol-1-yl)methyl)-3-cyclopropylphenyl)-3-(methoxymethoxy)isoxazole**

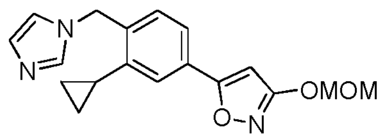

To a solution of **Compound 15C** (40 mg, 0.16 mmol), imidazole (10.9 mg, 0.16 mmol) and $K_2CO_3$ (44.3 mg, 0.32 mmol) in acetone (5 mL) was stirred at 50-60°C for 3 hours, cooled and filtered. The filtrate was poured into water (10 mL) and extracted with EtOAc (15 mL*3). The combined organic layers were dried over anhydrous $Na_2SO_4$, filtered and concentrated. The residue was purified by silica gel column chromatography to give **Compound 15D** (faint-yellow solid, 45 mg, 86.3% yield). LCMS (ESI) m/z: 326 (M+H+).

**Compound 15**

**5-(4-((1*H*-imidazol-1-yl)methyl)-3-cyclopropylphenyl)isoxazol-3-ol**

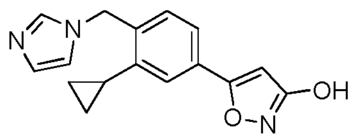

To a solution of **Compound 15D** (45 mg, 0.14 mmol) in MeOH (5 mL) was added dropwise 4N HCl-MeOH (1 mL) at 0°C. The mixture was stirred at 20°C for 0.5 hour and concentrated under reduced pressure. The residue was purified by prep-HPLC to give hydrochloride salt of **Compound 15** (8 mg, 21% yield). $^1H$ NMR (400MHz, METHANOL-$d_4$): δ 9.04 (s, 1H), 7.70 (d, *J* = 7.8 Hz, 1H), 7.64 (s, 2H), 7.55 (s, 1H), 7.39 (d, *J* = 8.0 Hz, 1H), 6.42 (s, 1H), 5.74 (s, 2H), 2.04-1.93 (m, 1H), 1.07- 0.98 (m, 2H), 0.79-0.71 (m, 2H). LCMS (ESI) m/z: 282 (M+H+).

**Example 16**

**5-(4-((1*H*-imidazol-1-yl)methyl)-3-ethylphenyl)isoxazol-3-ol**

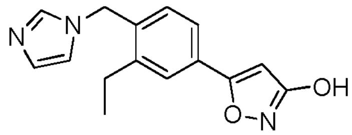

**Compound 16**

Synthesized by the same way as **Example 15.** $^1$H NMR (400MHz, METHANOL-$d_4$): δ 9.03 (s, 1H), 7.77 (s, 1H), 7.74-7.68 (m, 1H), 7.63 (d, *J* = 7.5 Hz, 2H), 7.34 (d, *J* = 8.0 Hz, 1H), 6.42 (s, 1H), 5.60 (s, 2H), 2.79 (q, *J* = 7.7 Hz, 2H), 1.23 (t, *J* = 7.7 Hz, 3H). LCMS (ESI) m/z: 270 (M+H$^+$).

**Scheme C**

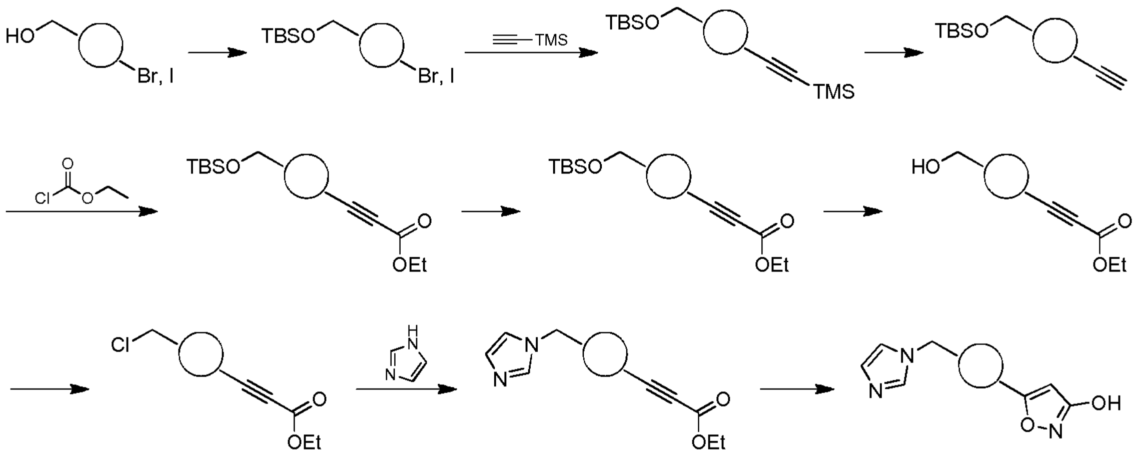

**Example 17**

**5-(4-((1*H*-imidazol-1-yl)methyl)-3,5-difluorophenyl)isoxazol-3-ol**

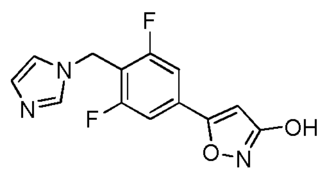

**Compound 17A**

**((4-bromo-2,6-difluorobenzyl)oxy)(*tert*-butyl)dimethylsilane**

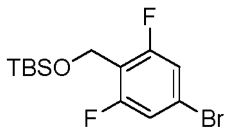

A mixture of (4-bromo-2,6-difluorophenyl)methanol (7.5 g, 33.5 mmol), TBDMSCl (10.8 g, 67.0 mmol) and imidazole (3.0 g, 40.2 mmol) in DMF (70 mL) was stirred at 20°C under $N_2$ atmosphere for 2 hours, quenched with water (350 mL) and extracted with EtOAc (200 mL*3). The combined organic layers were dried over anhydrous $Na_2SO_4$, filtered and concentrated. The residue was purified by silica gel column chromatography to give **Compound 17A** (colorless oil, 10.2 g, 90% yield). LCMS (ESI) m/z: 338 ($M+H^+$).

**Compound 17B**

***tert*-butyl((2,6-difluoro-4-((trimethylsilyl)ethynyl)benzyl)oxy)dimethylsilane**

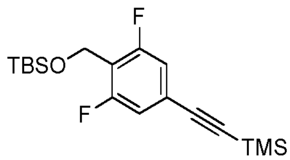

A mixture of **Compound 17A** (10.0 g, 29.6 mmol), ethynyltrimethylsilane (14.5 g, 89.0 mmol), triethylamine (9.0 g, 89.0 mmol), $PPh_3$ (777 mg, 2.96 mmol), CuI (564 mg, 2.96 mmol) and $Pd(PPh_3)Cl_2$ (1.0 g, 1.48 mmol) in THF (100 mL) was stirred at 80°C under $N_2$ atmosphere for 12 hours, cooled and filtered. The filtrate was poured into water (500 mL) and extracted with EtOAc (200 mL*5). The combined organic layers were dried over anhydrous $Na_2SO_4$, filtered and concentrated. The residue was purified by silica gel column chromatography to give **Compound 17B** (yellow oil, 8.0 g, 77% yield). LCMS (ESI) m/z: 355 ($M+H^+$).

**Compound 17C**

***tert*-butyl((2,6-difluoro-4-(ethynyl)-benzyl)oxy)dimethylsilane**

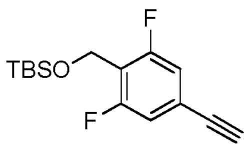

To a solution of **Compound 17B** (8.0 g, 22.5 mmol) in MeOH (80 mL) was added $K_2CO_3$ (9.3 g, 67.5 mmol) under $N_2$ atmosphere. The mixture was stirred at 20°C for 1 hours and filtered. The filtrate was poured into water (300 mL) and extracted with EtOAc (100 mL*6). The combined organic layers were dried over anhydrous $Na_2SO_4$, filtered and concentrated. The residue was purified by silica gel column chromatography to give **Compound 17C** (faint-yellow solid, 6.0 g, 95% yield). LCMS (ESI) m/z: 283 ($M+H^+$).

**Compound 17D**

**Ethyl 3-(4-(((*tert*-butyldimethylsilyl)oxy)methyl)-3,5-difluorophenyl)propiolate**

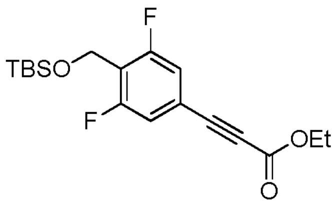

To a solution of **Compound 17C** (6.0 g, 21.2 mmol) in THF (50 mL) was added dropwise 2.5M *n*-BuLi (12.7 mL, 31.9 mmol) at -78°C under $N_2$ atmosphere. After being stirred for 1 hour, the solution of ethyl carbonochloridate (4.5 g, 42.4 mmol) in THF (10 mL) was added. The mixture was warmed to 20°C, stirred for another 2 hours, quenched with saturated $NH_4Cl$ solution (50 mL) and extracted with EtOAc (100 mL*5). The combined organic layers were dried over anhydrous $Na_2SO_4$, filtered and concentrated. The residue was purified by silica gel column chromatography to give **Compound 17D** (yellow oil, 5.8 g, 77% yield). LCMS (ESI) m/z: 355 ($M+H^+$).

**Compound 17E**

**Ethyl 3-(3,5-difluoro-4-(hydroxymethyl)phenyl)propiolate**

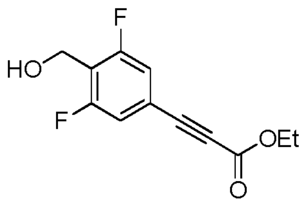

To a solution of **Compound 17D** (5.8 g, 16.4 mmol) in THF (50 mL) was added dropwise conc. HCl (6 mL) at 0°C. The mixture was stirred at 20°C for 12 hours, poured into water (250 mL) and extracted with EtOAc (100 mL *3). The combined organic layers were dried over anhydrous $Na_2SO_4$, filtered and concentrated under reduced pressure. The residue was purified by silica gel column chromatography to give **Compound 17E** (white solid, 3.9 g, 95% yield). LCMS (ESI) m/z: 241 ($M+H^+$).

**Compound 17F**

**Ethyl 3-(3,5-difluoro-4-(chloromethyl)-phenyl)propiolate**

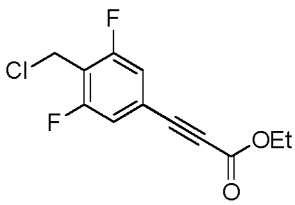

To a solution of **Compound 17E** (3.9 g, 16.2 mmol) and DMF (2.4 g) in DCM (20 mL) was added dropwise $SOCl_2$ (3.8 g, 32.4 mmol) at 0°C. After the addition, the mixture was stirred at 20°C for 2 hours, poured into water (50 mL) and extracted with DCM (50 mL *3). The combined organic layers were dried over anhydrous $Na_2SO_4$, filtered and concentrated under reduced pressure. The residue was purified by silica gel column chromatography to give **Compound 17F** (white solid, 3.0 g, 72% yield). LCMS (ESI) m/z: 259 ($M+H^+$).

**Compound 17G**

**Ethyl 3-(4-((1*H*-imidazol-1-yl)methyl)-3,5-difluorophenyl)propiolate**

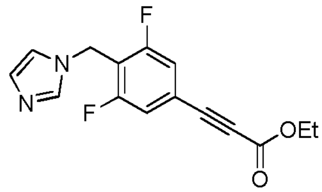

A solution of **Compound 17F** (3.0 g, 11.6 mmol), imidazole (1.6 g, 23.2 mmol), KI (0.96 g, 5.8 mmol) and $K_2CO_3$ (2.4 g, 17.4 mmol) in acetone (10 mL) was stirred at 50-60°C for 0.5 hour, cooled and filtered. The filtrate was poured into water (50 mL) and extracted with EtOAc (50 mL*5). The combined organic layers were dried over anhydrous $Na_2SO_4$, filtered and concentrated under reduced pressure. The residue was purified by silica gel column chromatography to give **Compound 17G** (faint-brown solid, 2.0 g, 60% yield). LCMS (ESI) m/z: 291 ($M+H^+$).

**Compound 17**

**5-(4-((1*H*-imidazol-1-yl)methyl)-3,5-difluorophenyl)isoxazol-3-ol**

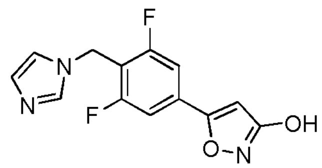

Solid NaOH (1.7 g, 41.4 mmol) was dissolved into water (8 mL) at 0°C. To this solution was added $NH_2OH{\cdot}HCl$ (1.4 g, 20.3 mmol) in batches. After being stirred for 10 mins, a solution of **Compound 17G** (2.0 g, 6.8 mmol) in MeOH (15 mL) was added dropwise to the mixture. The mixture was stirred at 20°C for 12 hours, acidized to pH = 8 by 2M HCl-MeOH solution and concentrated under reduced pressure to remove most of methanol and faint-yellow solid was precipitated . The residue was filtered. The faint-yellow filtered cake was collected and added into 4M HCl-MeOH (50 mL) to form a salt. The mixture was concentrated under reduced pressure and the residue was recrystalized in the mixed solvent of MeOH and EtOAc to give the hydrochloride salt of **Compound 17** (0.3 g, 16% yield). $^1H$ NMR (400MHz, METHANOL-$d_4$): δ 9.18 (br. s., 1H), 7.75-7.54 (m, 4H), 6.57 (s, 1H), 5.67 (s, 2H). LCMS (ESI) m/z: 278 ($M+H^+$).

**Example 18**

**5-(4-((1*H*-imidazol-1-yl)methyl)-3,5-dimethylphenyl)isoxazol-3-ol**

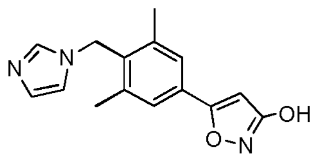

**Compound 18**

Synthesized by the same way as **Example 17.** $^1H$ NMR (400MHz, $CD_3OD$): δ 8.82 (s, 1H), 7.66-7.59 (m, 3H), 7.50 (s, 1H), 6.41 (s, 1H), 5.60 (s, 2H), 2.43 (s, 6H). LCMS (ESI) m/z: 270 ($M+H^+$).

**Example 19**

**5-(4-((1*H*-imidazol-1-yl)methyl)pyridin-2-yl)isoxazol-3-ol**

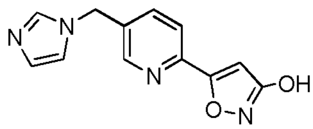

**Compound 19**

Synthesized by the same way as **Example 17.** $^1H$ NMR (400MHz, METHANOL-$d_4$): δ 9.18 (s, 1H), 8.78 (d, *J* = 1.5 Hz, 1H), 8.10-8.04 (m, 1H), 8.03-7.98 (m, 1H), 7.76 (s, 1H), 7.66 (s, 1H), 6.65 (s, 1H), 5.64 (s, 2H). LCMS (ESI) m/z: 243 ($M+H^+$).

**Scheme D**

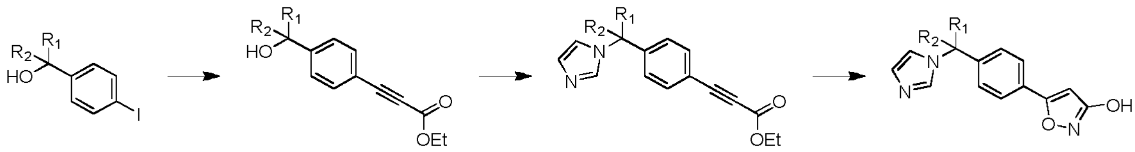

**Example 20**

**5-(4-(1-(1*H*-imidazol-1-yl)ethyl)phenyl)isoxazol-3-ol**

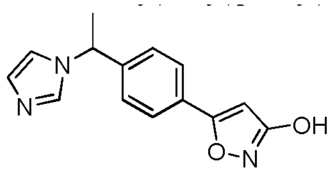

**Compound 20A**

**Ethyl 3-(4-(1-hydroxyethyl)phenyl)propiolate**

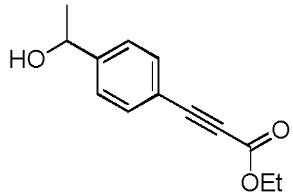

To a suspension of 1-(4-iodophenyl)methanol (10 g, 40.3 mmol), CuI (383 mg, 2.02 mmol) and $Pd(PPh_3)_2Cl_2$ (100 mg, 10% wt) in 1,4-dixoane (17 mL) was added a solution of ethyl propiolate (5.93 g, 60.4 mmol) in 1,4-dioxane (6 mL) under $N_2$ atmosphere. Then a solution of $Na_2CO_3$ (6.41 g, 60.5 mmol) in water (20 mL) was added to this mixture. After the addition, the mixture was stirred at 80 °C for 20 mins under $N_2$ atmosphere, poured into water (100 mL) and extracted with EtOAc (100 mL* 3). The combined organic layers were dried over anhydrous $Na_2SO_4$, filtered and con-

centrated. The residue was purified by silica gel column chromatography to give **Compound 20A** (colorless oil, 4.1 g, 46.6% yield). LCMS (ESI) m/z: 219 (M+H$^+$).

**Compound 20B**

**Ethyl 3-(4-(1-(1*H*-imidazol-1-yl)ethyl)phenyl)propiolate**

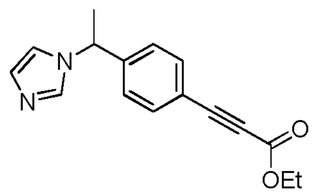

To a solution of **Compound 20A** (270 mg, 1.24 mmol), imidazole (101 mg, 1.48 mmol) and $PPh_3$ (389 mg, 1.48 mmol) in THF (5 mL) was added dropwise (*E*)-diisopropyl diazene-1,2-dicarboxylate (428 mg, 1.86 mmol) at 0 °C under $N_2$ atmosphere. After the addition, the mixture was stirred at 20 °C for 12 hours and concentrated under reduced pressure. The residue was purified by prep-TLC to give **Compound 20B** (brown oil, 120 mg, 36.2% yield). LCMS (ESI) m/z: 269 (M+H$^+$).

**Compound 20**

**5-(4-(1-(1*H*-imidazol-1-yl)ethyl)phenyl)isoxazol-3-ol**

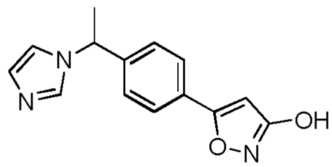

Solid NaOH (208 mg, 5.22 mmol) was dissolved into water (1.5 mL) at 0 °C. To this solution was added slowly $NH_2OH{\cdot}HCl$ (108 mg, 1.57 mmol) in batches. After being stirred for 10 mins, a solution of **Compound 20B** (140 mg, 0.52 mmol) in MeOH (1.5 mL) was added dropwise to the mixture. The mixture was stirred at 20°C for 12 hours, acidized to pH = 8 by 2N dilute HCl solution , and concentrated under reduced pressure to remove most of solvent. The residue was purified by prep-HPLC to give **Compound 20** (55 mg, 41.3% yield). $^1$H NMR (400 MHz, METHANOL-$d_4$): δ 7.81 (s, 1H), 7.69 (d, *J* = 8.16 Hz, 2H), 7.31 (d, *J* = 8.38 Hz, 2H), 7.18 (s, 1H), 6.99 (s, 1H), 6.13 (s, 1H), 5.56 (q, *J* = 7.06 Hz, 1H), 1.86 (d, *J* = 7.06 Hz, 3H). LCMS (ESI) m/z: 256 (M+H$^+$).

**Example 21 and 22**

**(*R*)-5-(4-(1-(1*H*-imidazol-1-yl)ethyl)phenyl)isoxazol-3-ol and (*S*)-5-(4-(1-(1*H*-imidazol-1-yl)ethyl)phenyl)isoxazol-3-ol**

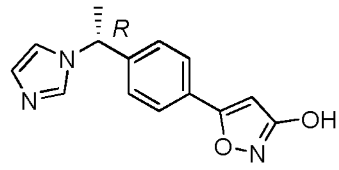
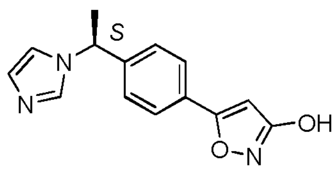

### Compound 21 and 22

The two examples were obtained from **Example 20** by SFC separation. The retention time of **Compound 21** was 5.198 min, and the retention time of **Compound 22** was 8.972 min. LCMS (ESI) m/z: 256 ($M+H^+$).

**Example 23**

**5-(4-(2-(1*H*-imidazol-1-yl)propan-2-yl)phenyl)isoxazol-3-ol**

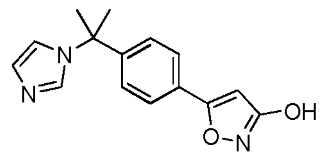

### Compound 23

Synthesized by the same way as **Example 20.** $^1$H NMR (400MHz, METHANOL-$d_4$): δ 9.22 (s, 1H), 7.84 (d, *J* = 8.0 Hz, 2H), 7.66 (br s, 2H), 7.43 (d, *J* = 8.0 Hz, 2H), 6.40 (s, 1H), 2.12 (s, 6H). LCMS (ESI) m/z: 270 ($M+H^+$).

### Scheme E

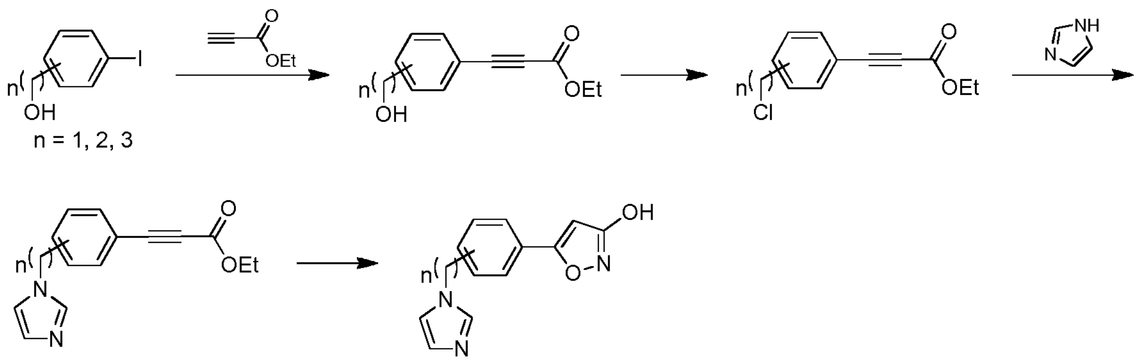


**Example 24**

**5-(3-((1*H*-imidazol-1-yl)methyl)phenyl)isoxazol-3-ol**

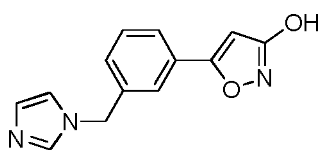

**Compound 24A**

**Ethyl 3-(3-(hydroxymethyl)phenyl)propiolate**

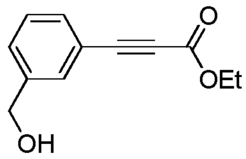

A mixture of (3-iodophenyl)methanol (5.9 g, 25.2 mmol), ethyl propiolate (5.0 g, 50.4 mmol) and $Cu_2O$ (3.6 g, 25.2 mmol) in DMF (100 mL) was stirred at 110 °C under $N_2$ atmosphere for 16 hours, cooled and filtered. The filtrate was concentrated under reduced pressure to remove most of DMF. The residue was added into water (100 mL) and extracted with EtOAc (100 mL*3). The combined organic layers were dried over anhydrous $Na_2SO_4$, filtered and concentrated. The crude product was purified by column chromatography to give **Compound 24A** (brown oil, 3.2 g, 62% yield). $^1H$ NMR (400 MHz, $CDCl_3$): δ 58 (s, 1 H), 7.49 (d, *J* = 7.50 Hz, 1 H), 7.40-7.46 (m, 1 H), 7.30-7.39 (m, 1 H), 4.68 (s, 2 H), 4.28 (q, *J* = 7.20 Hz, 2 H), 1.34 (t, *J* = 7.20 Hz, 3 H). LCMS (ESI) m/z: 205 ($M+H^+$).

**Compound 24B**

**Ethyl 3-(3-(chloromethyl)phenyl)propiolate**

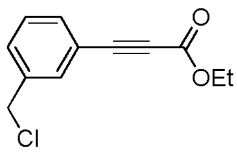

To a solution of **Compound 24A** (1.5 g, 7.3 mmol) and DMF (0.2 mL) in DCM (20 mL) was added dropwise $SOCl_2$ (2.6 g, 22 mmol) at 0 °C. After the addition, the mixture was stirred at 20 °C for 1 hour and then concentrated under reduced pressure to remove most of solvent and $SOCl_2$. The crude product **Compound 24B** (colorless oil, 1.6 g, 98% yield) was directly used for next step without further purification. LCMS (ESI) m/z: 223 ($M+H^+$).

**Compound 24C**

**Ethyl 3-(3-((1*H*-imidazol-1-yl)methyl)phenyl)propiolate**

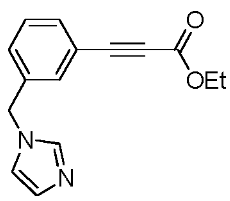

A mixture of **Compound 24B** (1.6 g, 7.2 mmol), imidazole (0.98 g, 14.4 mmol), KI (1.8 g, 10.8 mmol) and $K_2CO_3$ (2.0 g, 14.4 mmol) in acetone (50 mL) was stirred at 50-60 °C for 3 hours, cooled and filtered. The filtrate was poured into water (250 mL) and extracted with EtOAc (200 mL*3). The combined organic layers were dried over anhydrous $Na_2SO_4$, filtered and concentrated. The residue was purified by silica gel column chromatography to give **Compound 24C** (brown oil, 1.1 g, 59% yield). $^1H$ NMR (400 MHz, $CD_3OD$): δ 7.77 (s, 1 H), 7.54 (d, *J* = 7.50 Hz, 1 H), 7.39-7.48 (m, 2 H), 7.28-7.39 (m, 1 H), 7.13 (s, 1 H), 7.00 (s, 1 H), 5.25 (s, 2 H), 4.25 (q, *J* = 7.06 Hz, 2 H), 1.30 (t, *J* = 7.06 Hz, 3 H). LCMS (ESI) m/z: 255 ($M+H^+$).

**Compound 24**

**5-(3-((1*H*-imidazol-1-yl)methyl)phenyl)isoxazol-3-ol**

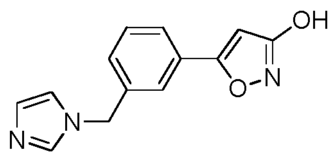

Solid NaOH (314 mg, 7.9 mmol) was dissolved into water (3 mL) at 0 °C. To this solution was added $NH_2OH \cdot HCl$ (164 mg, 2.4 mmol) in batches. After being stirred for 10 mins, a solution of **Compound 24C** (200 mg, 0.79 mmol) in MeOH (3 mL) was added dropwise to the mixture. The mixture was stirred at 20 °C for 3 hours and concentrated under reduced pressure to remove most of solvent. The residue was purified by prep-HPLC to give the hydrochloride salt of **Compound 24** (70 mg, 37% yield). $^1H$ NMR (400 MHz, $CD_3OD$): δ 9.17 (s, 1 H), 7.91 (s, 1 H), 7.83 (s, 1 H), 7.72 (s, 1 H), 7.50-7.66 (m, 3 H), 6.44 (s, 1 H), 5.58 (s, 2 H). LCMS (ESI) m/z: 242 ($M+H^+$).

**Scheme F**

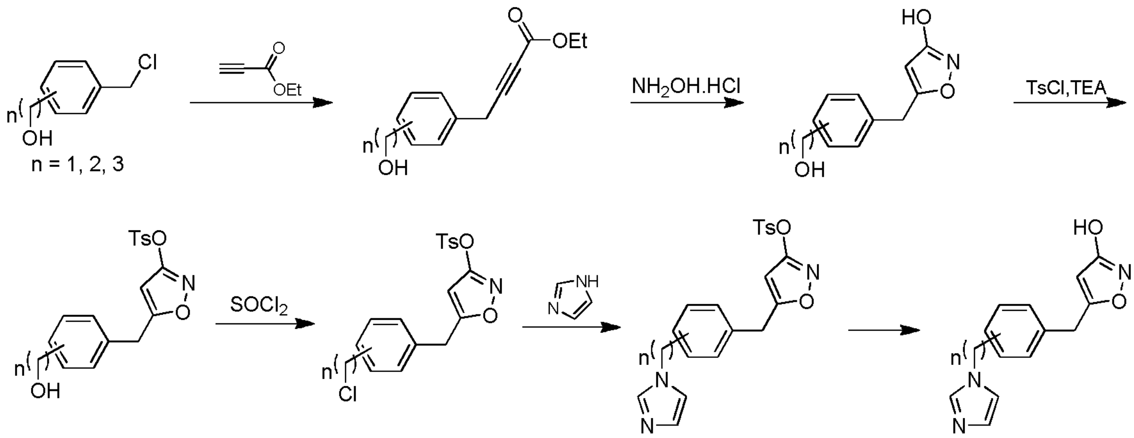


**Example 25**

**5-(4-(2-(1*H*-imidazol-1-yl)methyl)benzyl)isoxazol-3-ol**

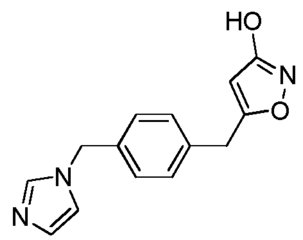

**Compound 25A**

**Ethyl 4-(4-(hydroxymethyl)phenyl)but-2-ynoate**

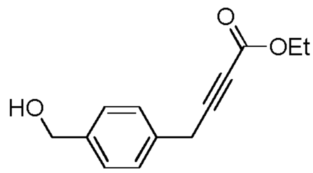

To a solution of (4-(chloromethyl)phenyl)methanol (4.50 g, 28.7 mmol) in MeCN (100 mL) was successively added ethyl propiolate (5.64 g, 57.5 mmol), CuI (5.47 g, 28.7 mmol), $K_2CO_3$ (3.97 g, 28.7 mmol) and tetrabutylammonium iodide (10.6 g, 28.7 mmol) at 15 °C under $N_2$ atmosphere. After the addition, the mixture was stirred at 50 °C under $N_2$ atmosphere for 12 hours, diluted with water (300 mL) and extracted with EtOAc (200 mL*3). The combined organic layers were dried over anhydrous $Na_2SO_4$, filtered and concentrated. The residue was purified by column chromatography to give **Compound 25A** (colorless oil, 4.7 g, 75.0% yield). LCMS (ESI) m/z: 219 (M+H+).

**Compound 25B**

**5-(4-(hydroxymethyl)benzyl)isoxazol-3-ol**

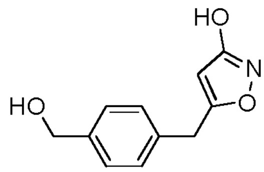

Solid NaOH (4.31 g, 107.7 mmol) was dissolved into water (25 mL) at 0 °C. To this solution was added a solution of $NH_2OH \cdot HCl$ (4.49 g, 64.6 mmol) in MeOH (50 mL) in batches. After being stirred for 10 mins, a solution of **Compound 25A** (4.7 g, 21.5 mmol) in MeOH (50 mL) was added dropwise to this mixture. The mixture was stirred at 20 °C for 12 hours and concentrated under reduced pressure to remove most of MeOH. The residue was acidized to pH = 2 by 3M HCl aqueous solution and extracted with EtOAc (100 mL*3). The combined organic layers were dried over anhydrous $Na_2SO_4$, filtered and concentrated. The residue was purified by column chromatography to give **Compound 25B** (2.2 g, 49.8% yield). LCMS (ESI) m/z: 206 (M+H+).

**Compound 25C**

**5-(4-(hydroxymethyl)benzyl)isoxazol-3-yl 4-methylbenzenesulfonate**

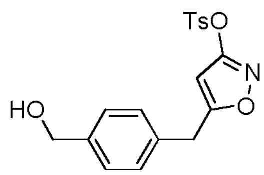

To a solution of **Compound 25B** (100 mg, 0.49 mmol) in DCM (3 mL) was successively added TosCl (557 mg, 2.92 mmol), triethylamine (394 mg, 3.90 mmol) and DMAP (6.0 mg, 0.05 mmol) at 15 °C under $N_2$ atmosphere. After the addition, the mixture was stirred at 15 °C for 5 hours under $N_2$ atmosphere and concentrated under reduced pressure. The residue was purified by column chromatography to give **Compound 25C** (yellow oil, 100 mg, 57.1% yield). LCMS (ESI) m/z: 360 (M+H+).

**Compound 25D**

**5-(4-(chloromethyl)benzyl)isoxazol-3-yl 4-methylbenzenesulfonate**

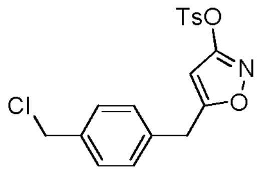

To a solution of **Compound 25C** (100 mg, 0.28 mmol) in DCM (3 mL) was added $SOCl_2$ (66 mg, 0.56 mmol) at 15 °C. After being stirred at 15 °C for 3 hours, the mixture was quenched by saturated $NaHCO_3$ solution (15 mL) and extracted with EtOAc (15 mL*3). The combined organic layers were dried over anhydrous $Na_2SO_4$, filtered and concentrated to give **Compound 25D** (yellow solid, 100 mg, 95.1% yield). LCMS (ESI) m/z: 378 ($M+H^+$).

**Compound 25E**

**5-(4-(choloromethyl)benzyl)isoxazol-3-yl 4-methylbenzenesulfonate**

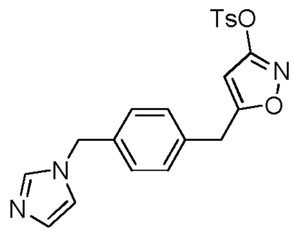

A mixture of **Compound 25D** (100 mg, 0.26 mmol), imidazole (36 mg, 0.53 mmol), KI (44 mg, 0.26 mmol) and $K_2CO_3$ (73 mg, 0.53 mmol) in acetone (2 mL) was stirred at 50 - 60 °C for 3 hours, cooled and filtered. The filtrate was poured into water (20 mL) and extracted with EtOAc (15 mL*3). The combined organic layers were dried over anhydrous $Na_2SO_4$, filtered and concentrated to give crude **Compound 25E** (yellow solid, 100 mg). LCMS (ESI) m/z: 410 ($M+H^+$).

**Compound 25**

**5-(4-(2-(1*H*-imidazol-1-yl)methyl)benzyl)isoxazol-3-ol**

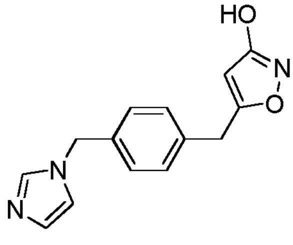

To a mixture of **compound 25E** (100 mg, 0.24 mmol) in water (2 mL) and MeOH (2 mL) was added 1M NaOH aqueous solution (1 mL, 1.0 mmol) at 15 °C. The mixture was stirred at 15 °C for 12 hours and concentrated under reduced pressure. The residue was purified by prep-HPLC to give the hydrochlorid salt of **Compound 25** (20 mg, 32.1% yield). $^1H$ NMR (400 MHz,$CD_3OD$): δ 9.07 (s, 1H), 7.63 (s, 1H), 7.58 (s, 1H), 7.32-7.44 (m, 4H), 5.70 (s, 1H), 5.45 (s, 2H), 4.01 (s, 2H). LCMS (ESI) m/z: 256 ($M+H^+$).

**Example 26**

**5-(3-((1*H*-imidazol-1-yl)methyl)benzyl)isoxazol-3-ol**

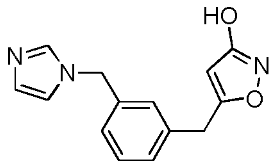

**Compound 26**

Synthesized by the same way as **Example 25.** $^1H$ NMR (400 MHz,$CD_3OD$): δ 9.06 (s, 1H), 7.63 (s, 1H), 7.58 (s, 1H), 7.38-7.46 (m, 1H), 7.29-7.38 (m, 3H), 5.69 (s, 1H), 5.45 (s, 2H), 4.02 (s, 2H). LCMS (ESI) m/z: 256 (M+1).

**Scheme G**

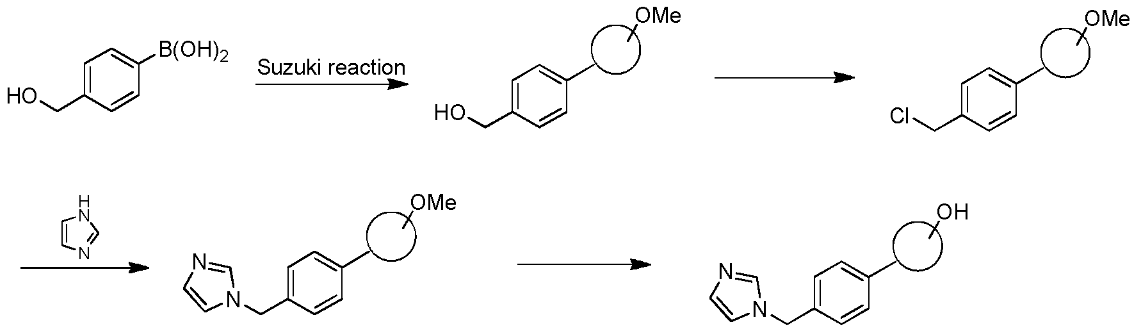


**Example 27**

**4-(4-((1*H*-imidazol-1-yl)methyl)phenyl)-3-fluoropyridin-2-ol**

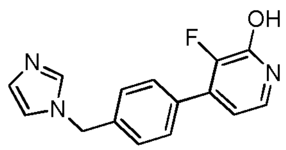

**Compound 27A**

**(4-(3-fluoro-2-methoxypyridin-4-yl)phenyl)methanol**

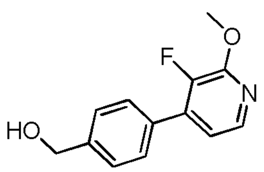

A mixture of 3-fluoro-4-iodo-2-methoxypyridine (2.5 g, 9.8 mmol), 4-(hydroxymethyl)phenyl)boronic acid (3.0 g, 19.6 mmol), $K_3PO_4$ (6.0 g, 29.6 mmol) and Pd(dppf)$Cl_2$ (0.5 g, 0.98 mmol) in 1,4-dioxane (18 mL) and water (3 mL)

was stirred at 90 °C under $N_2$ atmosphere for 12 hours. After cooled to room temperature, the mixture was diluted with water (60 mL) and extracted with EtOAc (50 mL*4). The combined organic layers were dried over anhydrous $Na_2SO_4$, filtered and concentrated under reduced pressure. The residue was purified by silica gel column chromatography to give **Compound 27A** (brown solid, 2.0 g, 87% yield). LCMS (ESI) m/z: 234 ($M+H^+$).

**Compound 27B**

**4-(4-(chloromethyl)phenyl)-3-fluoro-2-methoxypyridine**

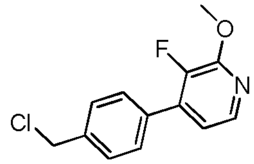

To a solution of **Compound 27A** (500 mg, 2.1 mmol) in DCM (5 mL) was successively added $SOCl_2$ (765 mg, 6.3 mmol) and DMF (450 mg, 6.3 mmol) at 0 °C under $N_2$ atmosphere. The mixture was stirred at 20 °C for 2 hours, poured into water (25 mL) and extracted with EtOAc (50 mL*3). The combined organic layers were dried over anhydrous $Na_2SO_4$, filtered and concentrated under reduced pressure. The residue was purified by column chromatography to give **Compound 27B** (white solid, 200 mg, 37% yield). LCMS (ESI) m/z: 252 ($M+H^+$).

**Compound 27C**

**4-(4-((1*H*-imidazol-1-yl)methyl)phenyl)-3-fluoro-2-methoxypyridine**

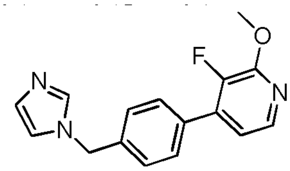

A mixture of **Compound 27B** (800 mg, 3.2 mmol), imidazole (432 mg, 6.4 mmol), KI (105 mg, 0.64 mmol) and $K_2CO_3$ (870 mg, 6.4 mmol) in acetone (10 mL) was stirred at 50 - 60 °C for 0.5 hour, cooled and filtered. The filtrate was poured into water (40 mL) and extracted with EtOAc (50 mL*3). The combined organic layers were dried over anhydrous $Na_2SO_4$, filtered and concentrated. The residue was purified by column chromatography to give **Compound 27C** (white solid, 700 mg, 77% yield). LCMS (ESI) m/z: 284 ($M+H^+$).

**Compound 27**

**4-(4-((1*H*-imidazol-1-yl)methyl)phenyl)-3-fluoropyridin-2-ol**

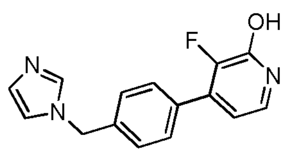

To a solution of **Compound 27C** (400 mg, 1.4 mmol) in MeOH (5 mL) was added 4M HCl-MeOH (20 mL) under $N_2$ atmosphere. The mixture was stirred at 20 °C for 12 hours and concentrated under reduced pressure. The residue was added into water (2 mL) and MeOH (3 mL), stirred for 10 mins and filtered. The filtered cake was purified by prep-HPLC to give the hydrochloride salt of **Compound 27** (100 mg, 26% yield). $^1$H NMR (400MHz, METHANOL-$d_4$): δ 9.13 (s, 1H), 7.74-7.66 (m, 3H), 7.64-7.51 (m, 3H), 7.34 (d, *J* = 6.5 Hz, 1H), 6.50 (t, *J* = 6.5 Hz, 1H), 5.55 (s, 2H). LCMS (ESI) m/z: 270 (M+1).

**Example 28**

**4'-((1*H*-imidazol-1-yl)methyl)-2,4-difluoro-[1,1'-biphenyl]-3-ol**

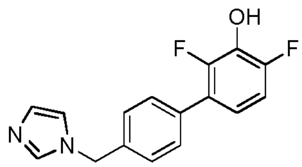

**Compound 28**

Synthesized by the same way as **Example 27.** $^1$H NMR (METHANOL-$d_4$, 400MHz): δ 9.11 (br. s., 1H), 7.69 (t, *J* = 1.8 Hz, 1H), 7.57-7.64 (m, 3H), 7.48-7.54 (m, 2H), 6.97-7.05 (m, 1H), 6.90 (td, *J* = 8.4, 5.8 Hz, 1H), 5.53 (s, 2H). LCMS (ESI) m/z: 287 (M+H$^+$).

**Example 29**

**5-(4-((1*H*-imidazol-1-yl)methyl)phenyl)isothiazol-3-ol**

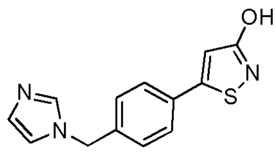

**Compound 29**

Synthesized by the same way as **Example 27.** $^1$H NMR (400 MHz, $CD_3OD$): δ 79.14 (s, 1H), 7.77-7.67 (m, 3H), 7.63 (s, 1H), 7.54 (d, *J* = 8.0 Hz, 2H), 6.91 (s, 1H), 5.55 (s, 2H). LCMS (ESI) m/z: 258 (M+H$^+$).

**Scheme H**

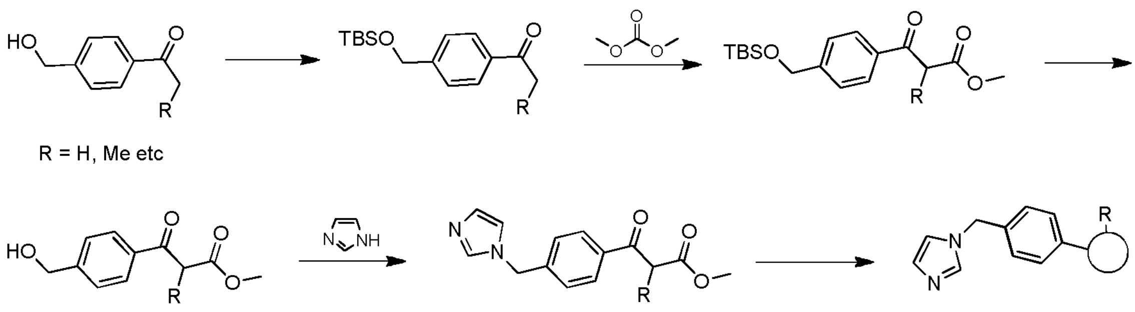


**Example 30**

**5-(4-((1*H*-imidazol-1-yl)methyl)phenyl)-4-methylisoxazol-3-ol**

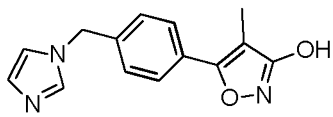

**Compound 30A**

**1-(4-(((*tert*-butyldimethylsilyl)oxy)methyl)phenyl)propan-1-one**

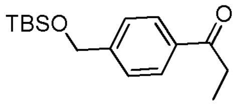

To a solution of 1-(4-(hydroxymethyl)phenyl)propan-1-one (3.2 g, 19.5 mmol) and imidazole (2.65 g, 39.0 mmol) in DMF (50 mL) was added tert-butyldimethylsilyl chloride (4.4 g, 29.2 mmol) at 0 °C under $N_2$ atmosphere. The mixture was stirred at 20 °C for 9 hours, diluted with EtOAc (200 mL) and washed with water (200 mL*2). The organic layer was dried over anhydrous $Na_2SO_4$, filtered and concentrated under reduced pressure. The residue was purified by silica gel column chromatography to give **Compound 30A** (yellow oil, 4.9 g, 84% yield). LCMS (ESI) m/z: 279 ($M+H^+$).

**Compound 30B**

**Methyl 3-(4-(((*tert*-butyldimethylsilyl)oxy)methyl)phenyl)-2-methyl-3-oxo propanoate**

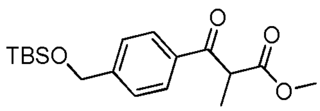

To a mixture of **Compound 30A** (4.9 g, 16.4 mmol) in dimethyl carbonate (70 mL) was added NaH (1.3 g, 32.8 mmol, 60% in mineral oil) in batches at 0 °C under $N_2$ atmosphere. The mixture was stirred at 90°C for 3 hours, cooled to room temperature, quenched by saturated $NH_4Cl$ aqueous solution (100 mL) and extracted with EtOAc (60 mL*3). The combined organic layers were dried over anhydrous $Na_2SO_4$, filtered and concentrated to give **Compound 30B** (yellow oil, 4.4 g, 80% yield). The crude product was directly used for next step without further purification. LCMS (ESI) m/z: 337 ($M+H^+$).

**Compound 30C**

**Methyl 3-(4-(hydroxymethyl)phenyl)-2-methyl-3-oxo propanoate**

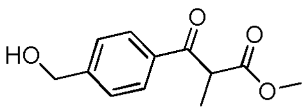

To a solution of **Compound 30B** (4.4 g, 13.1 mmol) in THF (30 mL) was added 1M HCl aqueous solution (30 mL) under $N_2$ atmosphere. The mixture was stirred at 25 °C for 3 hours, filtered and concentrated under reduced pressure. The residue was purified by silica gel column chromatography to give **Compound 30C** (yellow oil, 2.1 g, 72.4% yield). (LCMS (ESI) m/z: 223 ($M+H^+$).

**Compound 30D**

**Methyl 3-(4-((1*H*-imidazol-1-yl)methyl)phenyl)-2-methyl-3-oxo propanoate**

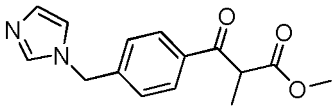

To a solution of **Compound 30C** (1.5 g, 6.75 mmol), imidazole (559 mg, 8.10 mmol) and tributylphosphine (2.69 g, 13.5 mmol) in anhydrous tetrahydrofuran (15 mL) was added dropwise (*E*)-di-*tert*-butyl diazene-1,2-dicarboxylate (3.1 g, 13.5 mmol) at 0°C under $N_2$ atmosphere. After the addition, the mixture was stirred at 20°C for 9 hours, diluted with EtOAc (200 mL) and washed with water (80 mL*2). The organic layer was dried over anhydrous $Na_2SO_4$, filtered and concentrated. The residue was purified by prep-HPLC to give **Compound 30D** (yellow oil, 150 mg, 8.1% yield). LCMS (ESI) m/z: 273 ($M+H^+$).

**Compound 30**

**5-(4-((1*H*-imidazol-1-yl)methyl)phenyl)-4-methylisoxazol-3-ol**

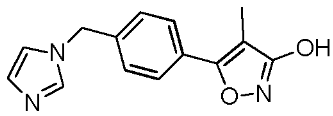

To a solution of **Compound 30D** (112 mg, 0.4 mmol) in MeOH (3 mL) was added a solution of NaOH (64 mg, 1.6 mmol) in water (1 mL) at -30°C. After being stirred for 10 mins, a solution of $NH_2OH{\cdot}HCl$ (56.0 mg, 0.8 mmol) in MeOH (2 mL) was added dropwise to this mixture. The resulting mixture was stirred at 20°C for 2 hours and concentrated under reduced pressure. The residue was purified by prep-HPLC to give the hydrochloride salt of **Compound 30** (50 mg, 50.1% yield). $^1H$ NMR (400 MHz, Methanol-*d*4): δ 9.09 (s, 1H), 7.63-7.73 (m, 3H), 7.58 (t, *J* = 7.83 Hz, 3H), 5.54 (s, 2H), 1.91-1.99 (m, 3H). LCMS (ESI) m/z: 256 ($M+H^+$).

**Example 31**

**5-(4-((1*H*-imidazol-1-yl)methyl)phenyl)-1*H*-pyrazol-3-ol**

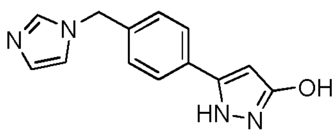

**Compound 31**

Synthesized by the same way as **Example 30.** $NH_2OH{\cdot}HCl$ was replaced by hydrazine hydrate. $^1H$ NMR ($CDCl_3$, 400MHz): δ 9.18 (s, 1H), 7.86- 7.88 (m, 3H), 7.64-7.71 (m, 3H), 6.35 (s, 1H), 5.60 (s, 2H). LCMS (ESI) m/z: 241 ($M+H^+$).

**Scheme I**

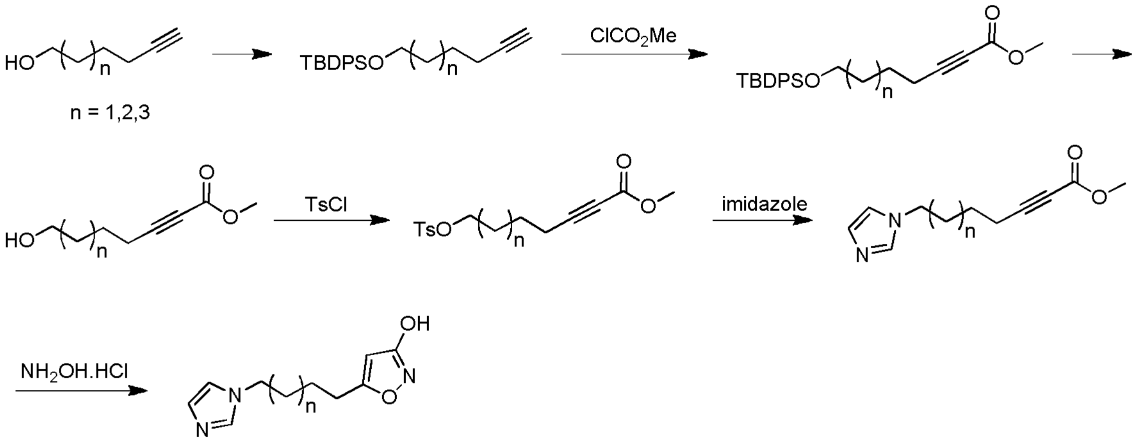


**Example 32**

**5-(4-(1*H*-imidazol-1-yl)butyl)isoxazol-3-ol**

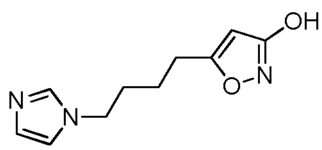

**Compound 32A**

***tert*-butyl(hex-5-yn-1-yloxy)diphenylsilane**

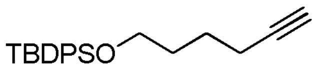

Synthesized by the same way as **Compound 17A.** TBSCl was replaced by TBDPSCl. LCMS (ESI) m/z: 337 (M+H+).

**Compound 32B**

**Methyl 7-((tert-butyldiphenylsilyl)oxy)hept-2-ynoate**

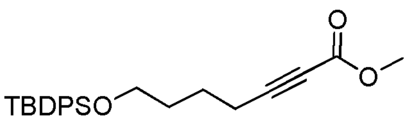

Synthesized by the same way as **Compound 17D.** Ethyl carbonochloridate was replaced by methyl carbonochloridate. LCMS (ESI) m/z: 395 (M+1).

**Compound 32C**

**Methyl 7-hydroxyhept-2-ynoate**

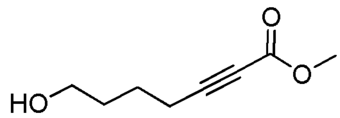

To a solution of **Compound 32B** (5.0 g, 12.6 mmol) in THF (50 mL) was added TBAF (4.0 g, 12.6 mmol) under $N_2$ atmosphere. The mixture was stirred at 20°C for 3 hours, poured into water (60 mL) and extracted with EtOAc (100 mL*3). The combined organic layers were dried over anhydrous $Na_2SO_4$, filtered and concentrated. The residue was purified by silica gel column chromatography to give **Compound 32C** (colorless oil, 1.2 g, 61% yield). LCMS (ESI) m/z: 157 ($M+H^+$).

**Compound 32D**

**Methyl 7-(tosyloxy)hept-2-ynoate**

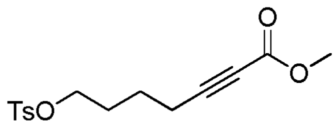

To a solution of **Compound 32C** (0.8 g, 5.12 mmol), triethylamine (1.13 g, 10.24 mmol) and DMAP (80 mg) in DCM (50 mL) was added TosCl (1.07 g, 5.63 mmol) in batches at 0°C under $N_2$ atmosphere. The mixtrure was stirred at 20°C for 1.5 hours, diluted with DCM (30 mL) and washed with water (50 mL*2). The organic layer was dried over anhydrous $Na_2SO_4$, filtered and concentrated. The residue was purified by silica gel column chromatography to give **Compound 32D** (yellow oil, 0.82 g, 78% yield). LCMS (ESI) m/z: 311 ($M+H^+$).

**Compound 32E**

**Methyl 7-(1*H*-imidazol-1-yl)hept-2-ynoate**

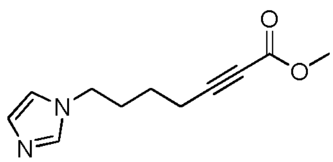

To a solution of imidazole (280 mg, 4.0 mmol) in DMF (5 mL) was added NaH (240 mg, 6.0 mmol, 60% in mineral oil) at 0°C under $N_2$ atmosphere. After being stirred for 10mins, **Compound 32D** (620 mg, 2.0 mmol) was added to this mixture. The mixture was stirred at 15°C for 5 hours, quenched by saturated $NH_4Cl$ aqueous solution (30 mL) and extracted with EtOAc (50 mL*3). The combined organic layers were dried over anhydrous $Na_2SO_4$, filtered and concentrated under reduced pressure. The residue was purified by prep-HPLC to give **Compound 32E** (yellow oil, 150 mg, 36% yield). LCMS (ESI) m/z: 207 ($M+H^+$).

**Compound 32**

**5-(4-(1*H*-imidazol-1-yl)butyl)isoxazol-3-ol**

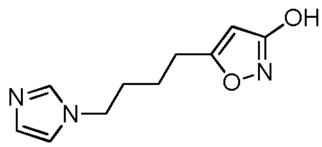

Synthesized by the same way as **Compound 25B.** $^1$H NMR (400 MHz, METHANOL-$d_4$): δ 9.04 (s, 1H), 7.72 (s, 1H), 7.65 (s, 1H), 3.60 (s, 1H), 2.10-2.40 (m, 2H), 2.20-2.50 (m, 2H), 1.70 - 2.00 (m, 4H). LCMS (ESI) m/z: 208 (M+H$^+$).

**Example 33**

**5-(5-(1*H*-imidazol-1-yl)pentyl)isoxazol-3-ol**

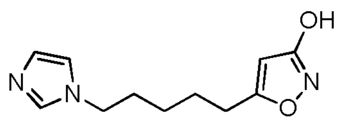

**Compound 33**

Synthesized by the same way as **Example 32.** $^1$H NMR (400MHz, METHANOL-$d_4$): δ 9.01 (s, 1H), 7.70 (s, 1H), 7.60 (s, 1H), 5.75 (s, 1H), 4.30 (t, *J*=7.3 Hz, 2H), 2.70 (t, *J*=7.3 Hz, 2H), 2.09 - 1.88 (m, 2H), 1.76 (q, *J*=7.7 Hz, 2H), 1.51 - 1.33 (m, 2H). LCMS (ESI) m/z: 222 (M+H$^+$).

**Scheme J**

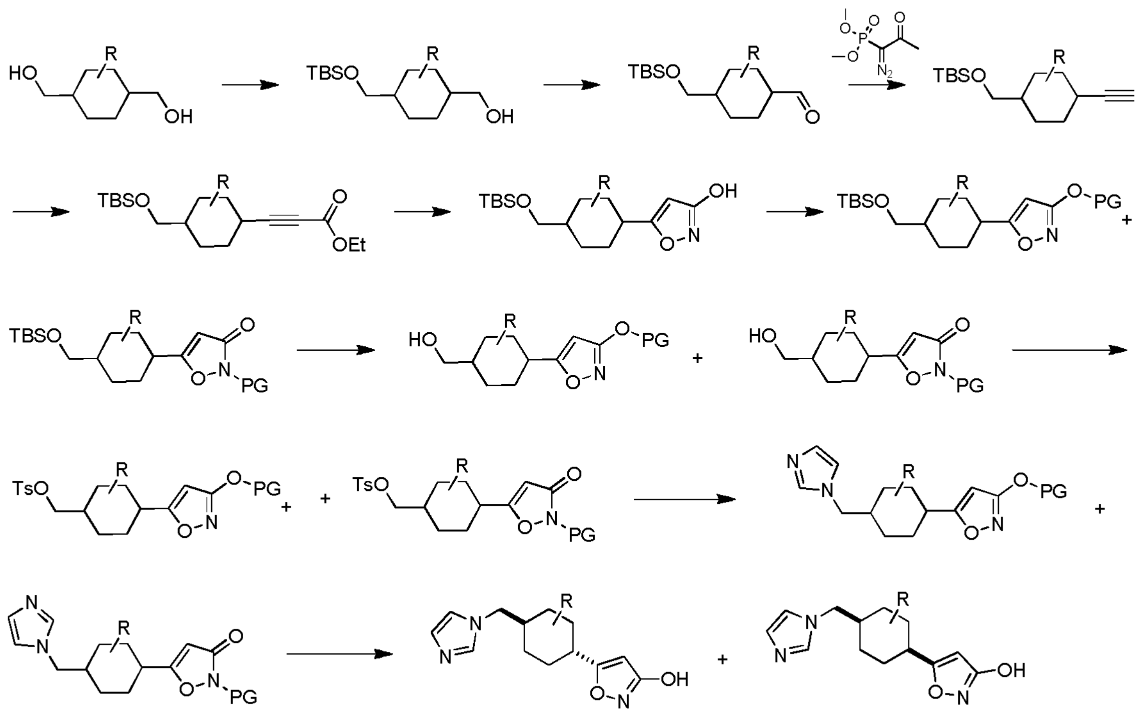

**Example 34&35**

**5-(*trans*-4-((1*H*-imidazol-1-yl)methyl)cyclohexyl)isoxazol-3-ol and 5-(*cis*-4-((1*H*-imidazol-1-yl)methyl)cyclohexyl)isoxazol-3-ol**

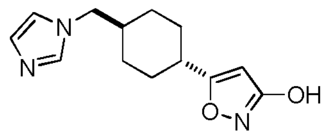

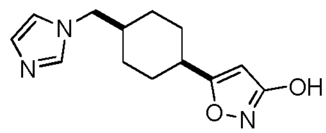

**Compound 34A&35A**

**(4-(((*tert*-butyldimethylsilyl)oxy)methyl)cyclohexyl)methanol**

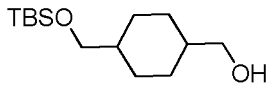

Synthesized by the same way as **Compound 17A.** LCMS (ESI) m/z: 259 ($M+H^+$).

**Compound 34B&35B**

**4-(((*tert*-butyldimethylsilyl)oxy)methyl)cyclohexane carbaldehyde**

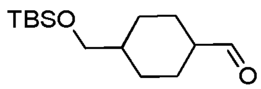

To a solution of **Compound 34A&35A** (12.9 g, 50.0 mmol) in DCM (350 mL) was added Dess-Martin reagent (21.5 g, 50.0 mmol) at 0°C under $N_2$ atmosphere. The mixture was stirred at 25°C for 3 hours and filtered. The filtrate was diluted with DCM (200 mL), successively washed with saturated $Na_2CO_3$ solution (50 mL) and water (50 mL), dried over anhydrous $Na_2SO_4$, filtered and concentrated under reduced pressure. The residue was purified by silica gel column chromatography to give **Compound 34B&35B** (yellow solid, 6.45 g, 50% yield). LCMS (ESI) m/z: 257($M+H^+$).

**Compound 34C&35C**

***tert*-butyl((4-ethynylcyclohexyl)methoxy)dimethylsilane**

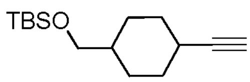

To a mixture of **Compound 34B&35B** (6.4 g, 25 mmol) and $K_2CO_3$ (6.98 g, 25 mmol) in MeOH (300 mL) was added dimethyl (1-azido-2-oxopropyl)phosphonate (4.78 g, 25 mmol) at 0°C under $N_2$ atmosphere. The mixture was stirred at 25°C for 9 hours, diluted with EtOAc (200 mL), washed with water (100 mL*2). The organic layer was dried over anhydrous $Na_2SO_4$, filtered and concentrated. The residue was purified by silica gel column chromatography to give **Compound 34C&35C** (yellow oil, 2.7 g, 42% yield). LCMS (ESI) m/z: 253 ($M+H^+$).

**Compound 34D&35D**

**Ethyl 3-(4-(((*tert*-butyldimethylsilyl)oxy)methyl)cyclohexyl)propiolate**

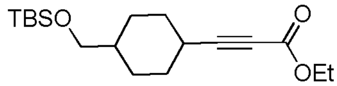

Synthesized by the same way as **Compound 17D.** LCMS (ESI) m/z: 325 ($M+H^+$).

**Compound 34E&35E**

**5-(4-(((*tert*-butyldimethylsilyl)oxy)methyl)cyclohexyl)isoxazol-3-ol**

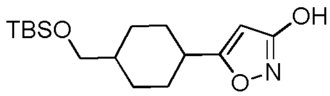

Synthesized by the same way as **Compound 25B.** LCMS (ESI) m/z: 312 ($M+H^+$).

**Compounds 34F&35F**

**5-(4-(((*tert*-butyldimethylsilyl)oxy)methyl)cyclohexyl)-3-((4-methoxybenzyl)oxy)isoxazole** and **5-(4-(((tert-butyldimethylsilyl)oxy)methyl)cyclohexyl)-2-(4-methoxybenzyl)isoxazol-3(2*H*)-one**

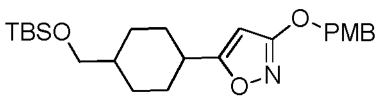 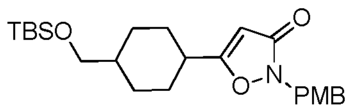

To a mixture of **Compound 34E&35E** (15.0 g, 48.2 mmol) and $K_2CO_3$ (13.3 g, 96.3 mmol) in DMF (8 mL) was added dropwise *p*-methoxybenzyl chloride (9.8 g, 62.6 mmol) under $N_2$ atmosphere. The mixture was stirred at 25°C for 5 hours, poured into water (150 mL) and washed with EtOAc (200 mL*3). The organic layer was dried over anhydrous $Na_2SO_4$, filtered and concentrated under reduced pressure. The residue was purified by silica gel column chromatography to give 5-(4-(((*tert*-butyldimethylsilyl)oxy)methyl)cyclohexyl)-3-((4-methoxybenzyl)oxy)isoxazole (yellow solid, 5.0 g, 24.1% yield) and 5-(4-(((*tert*-butyldimethylsilyl)oxy)methyl)cyclohexyl)-2- (4-methoxybenzyl)isoxazol-3 (2*H*)-one (yellow solid, 12.0 g, 57.7% yield). LCMS (ESI) m/z: 432 ($M+H^+$).

**Compounds 34G&35 G**

**(4-(3-((4-methoxybenzyl)oxy)isoxazol-5-yl)cyclohexyl)methanol and 5-(4-(hydroxymethyl)cyclohexyl)-2-(4-methoxybenzyl)isoxazol-3(2*H*)-one**

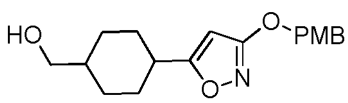 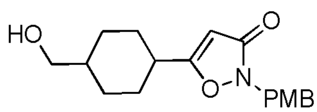

Synthesized by the same way as **Compound 17E.** LCMS (ESI) m/z: 318 ($M+H^+$).

**Compounds 34H&35H**

**(4-(3-((4-methoxybenzyl)oxy)isoxazol-5-yl)cyclohexyl)methyl 4-methylbenzenesulfonate and**

**(4-(2-(4-methoxybenzyl)-3-oxo-2,3-dihydroisoxazol-5-yl)cyclohexyl)methyl 4-methylbenzenesulfonate**

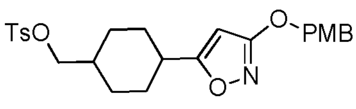
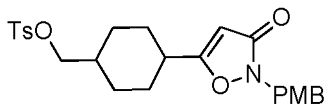

Synthesized by the same way as **Compound 32D.** LCMS (ESI) m/z: 471 ($M+H^+$).

**Compounds 34I&35I**

**5-(4-((1*H*-imidazol-1-yl)methyl)cyclohexyl)-3-((4-methoxybenzyl)oxy)isoxazole and 5-(4-((1*H*-imidazol-1-yl)methyl)cyclohexyl)-2-(4-methoxybenzyl)isoxazol-3(2*H*)-one**

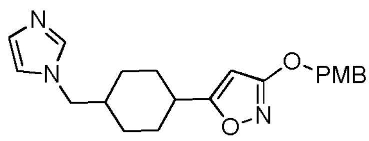
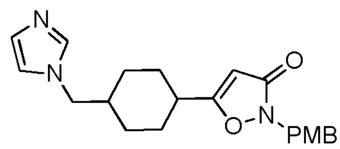

Synthesized by the same way as **Compound 17G.** LCMS (ESI) m/z: 368 ($M+H^+$).

**Compounds 34&35**

**5-(*trans*-4-((1*H*-imidazol-1-yl)methyl)cyclohexyl)isoxazol-3-ol and 5-(*cis*-4-((1*H*-imidazol-1-yl)methyl)cyclohexyl)isoxazol-3-ol**

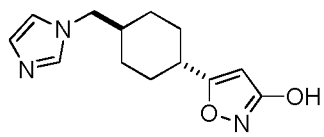
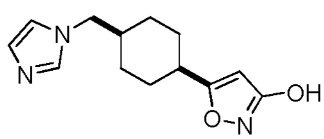

**Compounds 34I&35I**

**Compounds 34I&35I** (5-(4-((1*H*-imidazol-1-yl)methyl)cyclohexyl)-3-((4-methoxy benzyl)oxy)isoxazole, 2.0 g, 5.44 mmol and 5-(4-((1*H*-imidazol-1-yl)methyl) cyclohexyl)-2-(4-methoxybenzyl)isoxazol-3(2*H*)-one, 4.0 g, 10.8 mmol) were added into trifluoroacetic acid (48 mL) and methanesulfonic acid (6 mL) under $N_2$ atmosphere. The mixture was stirred at 100°C for 12 hours, poured into water (150 mL), basified to pH =6-7 by saturated $Na_2CO_3$ solution and filtered to collect the precipitate. The filtered cake was purified by prep-HPLC to give the hydrochloride salt of **Compound 34** (5-(*trans*-4-((1*H*-imidazol-1-yl)methyl)cyclohexyl) isoxazol-3-ol, 400 mg) and the hydrochloride salt of **Compound 35** (5-(*cis*-4-((1*H*-imidazol-1-yl)methyl)cyclohexyl)isoxazol-3-ol, 300 mg). Total yield was 17.5%. **Compound34:** $^1H$ NMR (400MHz, METHANOL-d4): δ 9.01 (s, 1H), 7.69 (s, 1H), 7.62 (s, 1H), 5.69 (s, 1H), 4.18 (d, *J* = 7.3 Hz, 2H), 2.76 - 2.61 (m, 1H), 2.12 (d, *J*=11.0 Hz, 2H), 1.97 (ttd, *J* = 3.8, 7.7, 15.2 Hz, 1H), 1.77 (d, *J*=11.5 Hz, 2H), 1.48 (dq, *J* = 3.0, 12.9 Hz, 2H), 1.33-1.14 (m, 2H). LCMS (ESI) m/z: 248 ($M+H^+$). **Compound 35:** $^1H$ NMR (400MHz, METHANOL-d4): δ 9.00 (s, 1H), 7.68 (s, 1H), 7.60 (s, 1H), 5.82 (s, 1H), 4.20 (d, *J*=7.5 Hz, 2H), 3.02 (t, *J*=4.8 Hz, 1H), 2.14 - 1.99 (m, 3H), 1.86 - 1.75 (m, 2H), 1.66 - 1.53 (m, 2H), 1.40 - 1.25 (m, 2H). LCMS (ESI) m/z: 248 ($M+H^+$).

## Scheme K

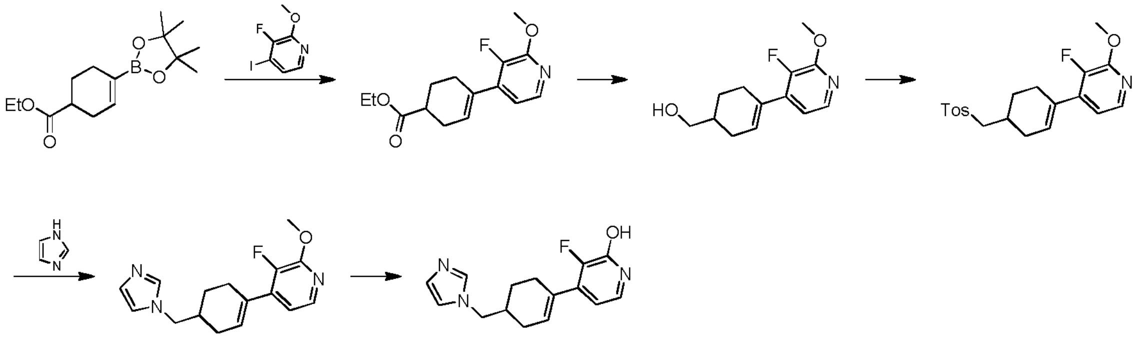

**Example 36**

**4-(4-((1*H*-imidazol-1-yl)methyl)cyclohex-1-en-1-yl)-3-fluoropyridin-2-ol**

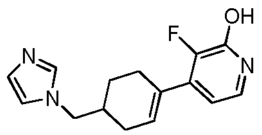

**Compound 36A**

**Ethyl 4-(3-fluoro-2-methoxypyridin-4-yl)cyclohex-3-enecarboxylate**

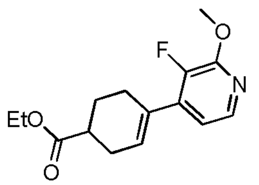

Synthesized by the same way as **Compound 27A.** LCMS (ESI) m/z: 280 ($M+H^+$).

**Compound 36B**

**(4-(3-fluoro-2-methoxypyridin-4-yl)cyclohex-3-en-1-yl)methanol**

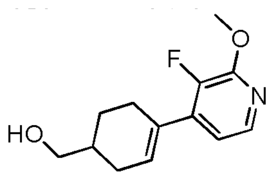

To a solution of **Compound 36A** (600 mg, 2.15 mmol) in THF (5 mL) was added $LiBH_4$ (234 mg, 10.7 mmol) under $N_2$ atmosphere. The mixture was stirred at 25°C for 12 hours, quenched by water (10 mL) and extracted with EtOAc (30 mL*3). The combined organic layers were dried over anhydrous $Na_2SO_4$, filtered and concentrated under reduced pressure. The residue was purified by silica gel column chromatography to give **Compound 36B** (white solid,

450 mg, 88.2% yield). LCMS (ESI) m/z: 238 ($M+H^+$).

**Compound 36C**

**3-fluoro-2-methoxy-4-(4-(tosylmethyl)cyclohex-1-en-1-yl)pyridine**

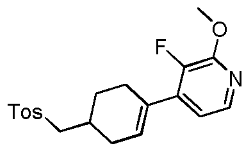

Synthesized by the same way as **Compound 32D.** LCMS (ESI) m/z: 376 ($M+H^+$).

**Compound 36D**

**4-(4-((1*H*-imidazol-1-yl)methyl)cyclohex-1-en-1-yl)-3-fluoro-2-methoxypyridine**

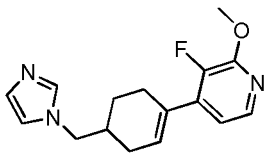

Synthesized by the same way as **Compound 17G.** LCMS (ESI) m/z: 288 ($M+H^+$).

**Compound 36**

**4-(4-((1H-imidazol-1-yl)methyl)cyclohex-1-en-1-yl)-3-fluoropyridin-2-ol**

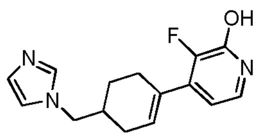

Synthesized by the same way as **Compound 27.** $^1$H NMR (400MHz, METHANOL-$d_4$)): $\delta$ 9.06 (s, 1H), 7.98 - 7.51 (m, 2H), 7.20 (d, *J* = 6.5 Hz, 1H), 6.31 (t, *J* =6.1 Hz, 1H), 6.16 (br. s., 1H), 4.29 (d, *J* =7.0 Hz, 2H), 2.66 - 2.19 (m, 4H), 2.12 - 1.77 (m, 2H), 1.59 - 1.42 (m, 1H). LCMS (ESI) m/z: 274 ($M+H^+$).

**Study Example 1:** Study for the release of TXB2 in the in-vitro coagulation process of rats' whole blood.

**Principle:**

In the in-vitro coagulation process, thromboxane A2 (TXA2) pathway was activated, resulting in the formation of large amounts of TXA2, which were rapidly metabolized to its stable form thromboxane B2 (TXB2).

The key enzyme of this pathway was thromboxane synthase. The generation of TXA2 could be inhibited by blocking this enzyme with specific inhibitors (tested compounds), and the level of TXB2 was thus decreased.

The activity of tested compound was measured with the $IC_{50}$ of inhibiting the formation of TBX2.

**Animals:**

Male Sprague-Dawley (SD) rats, purchased from Shanghai SLAC Laboratory Animal Co., Ltd.

**Reagents:**

0.9% normal saline, 100 M NaOH solution (vehicle), 100 uM thromboxane synthetase inhibitor solution, isoflurane

**Procedure and Method:**

1. Tested compound was dissolved by normal saline and prepared to required concentration by gradient dilution. 167 uL of prepared solution was added to centrifuge tube.

2. Whole blood was collected from the heart of male SD rat after isoflurane anesthesia, added to centrifuge tube (0.5 mL), shaken up and incubated under 37°C water bath for 30 mins until complete coagulation.

3. Centrifuge tube was taken out and centrifuged for 5 mins (6000g, 4°C) to get serum.

4. Assay the content of TXB2 by LC-MS/MS-AG (API 4000).

**Data and Analysis:**

By comparison with the vehicle group, TXB2 contents of each sample were normalized to Control% by the formule:

$$\text{Control\%} = (\text{TBX2 level in test tube} - \text{TBX2 level in negative tube}) / \text{TBX2 level in control tube} \times 100$$

Note: Test tube: solution of tested compound with series concentration
Negative tube: solution of thromboxane synthetase inhibitor at high concentration (100 uM)
Control tube: vehicle

Using GraphPad Prism (concentration as X-axis and Control% as Y-axis) to fit the data and calculate $IC_{50}$.

**The result was shown in table 1:**

Table 1 $IC_{50}$ for the release level of TXB2 in the in-vitro coagulation of rats' whole blood.

| Test sample (compounds) | thromboxane synthetase | Test sample (compounds) | thromboxane synthetase |
|---|---|---|---|
| Ozagrel | 120nM | Compound 19 | E |
| Compound 1 | A | Compound 20 | A |
| Compound 2 | D | Compound 21 | A |
| Compound 3 | C | Compound 22 | C |
| Compound 4 | B | Compound 23 | D |
| Compound 5 | C | Compound 24 | E |
| Compound 6 | B | Compound 25 | E |
| Compound 7 | C | Compound 26 | E |
| Compound 8 | C | Compound 27 | E |
| Compound 9 | A | Compound 28 | D |
| Compound 10 | C | Compound 29 | E |
| Compound 11 | B | Compound 30 | E |

(continued)

| Test sample (compounds) | thromboxane synthetase | Test sample (compounds) | thromboxane synthetase |
|---|---|---|---|
| Compound 12 | B | Compound 31 | E |
| Compound 13 | B | Compound 32 | E |
| Compound 14 | E | Compound 33 | E |
| Compound 15 | C | Compound 34 | B |
| Compound 16 | A | Compound 35 | C |
| Compound 17 | A | Compound 36 | D |
| Compound 18 | E | | |
| Note: A≤25nM; 25nM<B≤50nM; 50nM<C≤100nM; 100nM<D≤250nM; E>250nM | | | |

## Claims

1. A compound of formula (I), a pharmaceutically acceptable salt or a tautomer thereof,

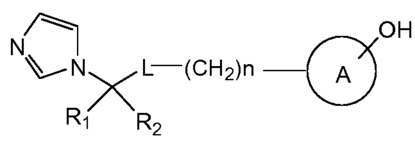

(I)

wherein,

n is an integer of 0 to 3, preferably 0 or 1;
L is selected from a 5- to 6- membered cyclohydrocarbyl or heterocyclohydrocarbyl or $-(CH_2)_{1-6}-$, each of which is optionally substituted by R;
ring A is selected from a 5- to 6- membered unsaturated cyclohydrocarbyl or heterocyclyl, each of which is optionally substituted by R;
each of $R_1$, $R_2$, R is independently selected from H, F, Cl, Br, I, CN, OH, SH, $NH_2$, CHO, COOH, or selected from $C(=O)NH_2$, $S(=O)NH_2$, $S(=O)_2NH_2$, an $C_{1-6}$ alkyl or heteroalkyl, a $C_{3-6}$ cycloalkyl or heterocycloalkyl, each of which is optionally substituted by $R_{01}$;
"hetero-" represents a heteroatom or a heteroatomic group, which is selected from -C(=O)N(R)-, -N(R)-, -C(=NR)-, $-S(=O)_2N(R)-$, -S(=O)N(R)-, -O-, -S-, -C(=O)O-, -C(=O)-, -C(=S)-, -S(=O)-, - $S(=O)_2-$ or -N(R)C(=O)N(R)-;
each of the number of $R_{01}$, the heteroatom or the heteroatomic group is independently selected from 0, 1, 2 or 3; and
$R_{01}$ is selected from H, F, Cl, Br, I, CN, OH, an $C_{1-3}$ alkyl, $N(CH_3)_2$, $NH(CH_3)$, $NH_2$, CHO, COOH, $C(=O)NH_2$, $S(=O)NH_2$, $S(=O)_2NH_2$, trifluoromethyl, aminomethyl, hydroxymethyl, methoxyl, formoxyl, methoxycarbonyl, methylsulfonyl, methylsulfinyl.

2. The compound, the pharmaceutically acceptable salt or the tautomer thereof according to claim 1, wherein, each of $R_1$, $R_2$, R is independently selected from H, F, Cl, Br, I, an $C_{1-3}$ alkyl, an $C_{1-3}$ alkoxy, $N(CH_3)_2$, $NH(CH_3)$, $NH_2$, CHO, COOH, $C(=O)NH_2$, $S(=O)NH_2$, $S(=O)_2NH_2$, trifluoromethyl, aminomethyl, hydroxymethyl, formoxyl, methoxycarbonyl, methylsulfonyl, methylsulfinyl, cyclopropyl;
preferably, each of $R_1$, $R_2$, R is independently selected from H, F, Cl, Br, I, $CH_3$, $C_2H_5-$, $CH_3O-$ or

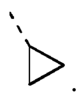

3. The compound, the pharmaceutically acceptable salt or the tautomer thereof according to claim 1 or 2, wherein, L

is selected from a 5- to 6- membered aryl or heteroaryl, a 5- to 6- membered aliphatic cyclohydrocarbyl, $-(CH_2)_{1-6}-$, each of which is optionally substituted by R;
preferably, L is selected from

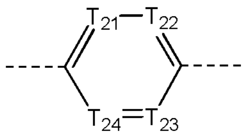 , 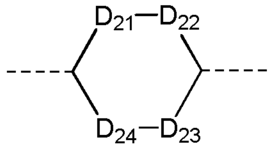 ,

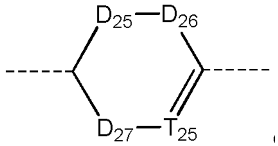

or $-(CH_2)_{1-6}-$ which is optionally substituted by R, wherein,
none or one of $T_{21-24}$ is N, and the others are C(R);
zero to three of $D_{21-24}$ are selected from -C(=O)N(R)-, -N(R)-, -C(=NR)-, $-S(=O)_2N(R)-$, - S(=O)N(R)-, -O-, -S-, -C(=O)O-, -C(=O) -, -C(=S)-, -S(=O)-, $-S(=O)_2-$ or -N(R)C(=O)N(R)-, and the others are C(R) (R);
$T_{25}$ is N or C(R);
zero to three of $D_{25-27}$ are selected from -C(=O)N(R)-, -N(R)-, -C(=NR)-, $-S(=O)_2N(R)-$, - S(=O)N(R)-, -O-, -S-, -C(=O)O-, -C(=O)-, -C(=S)-, -S(=O)-, $-S(=O)_2-$ or -N(R)C(=O)N(R)-, and the others are C(R) (R).

**4.** The compound, the pharmaceutically acceptable salt or the tautomer thereof according to claim 3, wherein, L is selected from

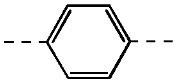 , 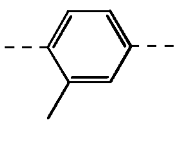 , 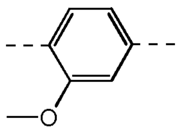 , 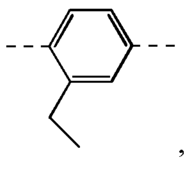 ,

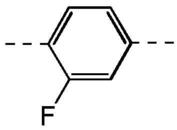 , 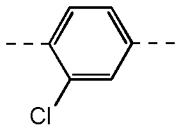 , 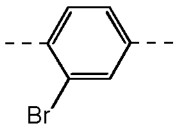 , 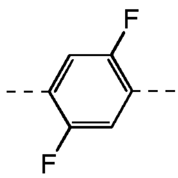 , 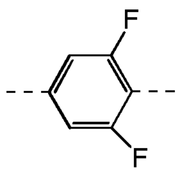 ,

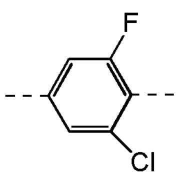 , 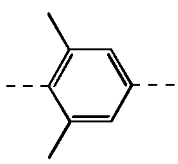 , 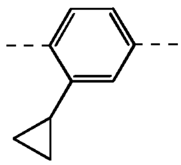 , 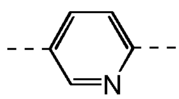 , 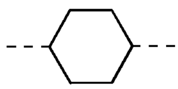 ,

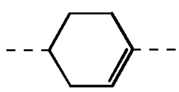 , 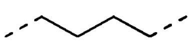 .

**5.** The compound, the pharmaceutically acceptable salt or the tautomer thereof according to claim 1 or 2, wherein, A is selected from a 5- to 6- membered aryl or heteroaryl; preferably, A is selected from

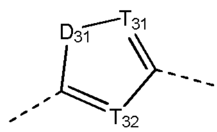, 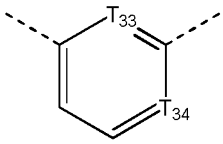;

each of $T_{31-34}$ is independently selected from N or C(R), $D_{31}$ is selected from -C(R)(R)-, -C(=O)N(R)-, -N(R)-, -C(=NR)-, $-S(=O)_2N(R)-$, -S(=O)N(R)-, - O-, -S-, -C(=O)O-, -C(=O)-, -C(=S)-, -S(=O)-, $-S(=O)_2-$ or -N(R)C(=O)N(R)-.

**6.** The compound, the pharmaceutically acceptable salt or the tautomer thereof according to claim 5, wherein, A is selected from

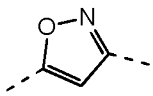, 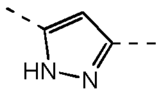, 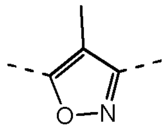, 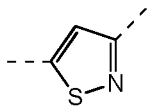,

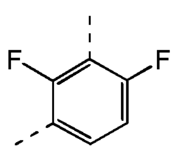, 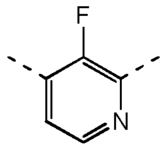.

**7.** The compound, the pharmaceutically acceptable salt or the tautomer thereof according to claim 5, wherein, the moiety

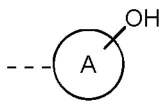

is selected from

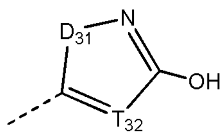, 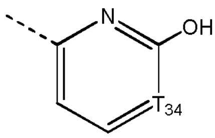,

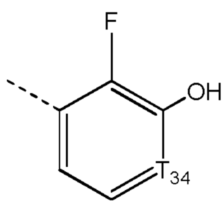;

the tautomer thereof is selected from

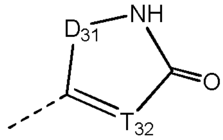,

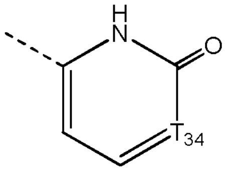.

8. The compound, the pharmaceutically acceptable salt or the tautomer thereof according to claim 6 or 7 , wherein, the moiety

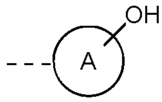

is selected from

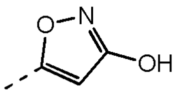, 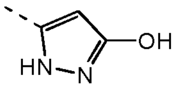,

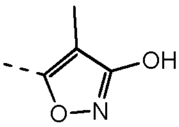, 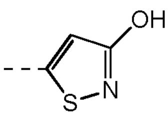, 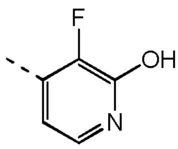, 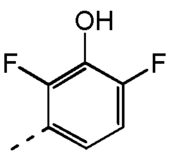;

the tautomer thereof is selected from

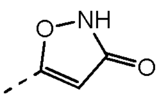, 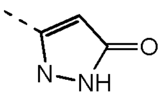, 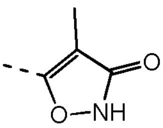, 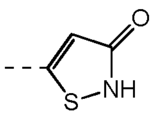, 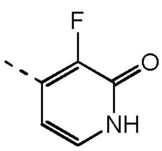.

9. The compound, the pharmaceutically acceptable salt or the tautomer thereof according to claim 1, which is **characterized in that**, the compound of formula (I) is selected from the group consisting of

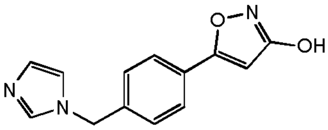 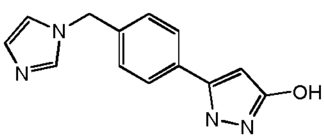 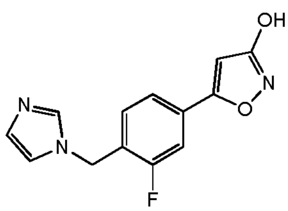

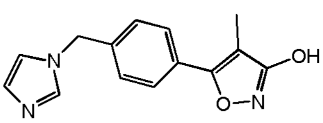 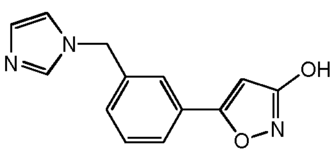 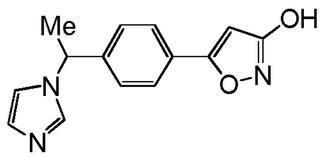

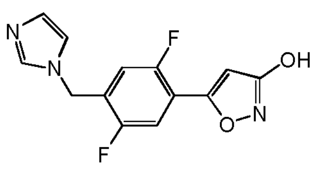
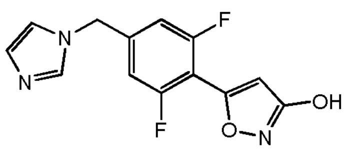
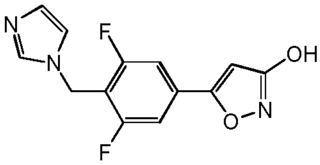

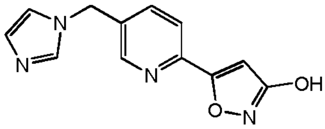
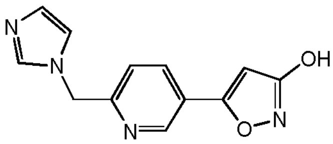
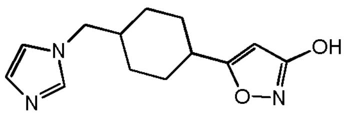

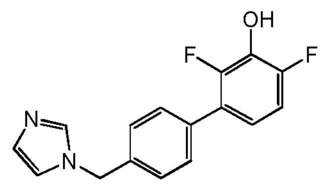
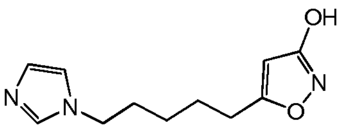
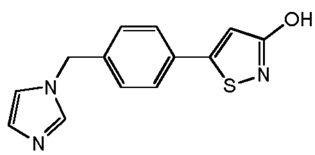

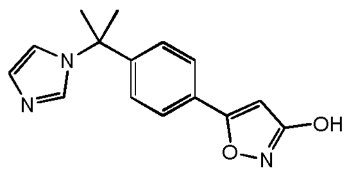
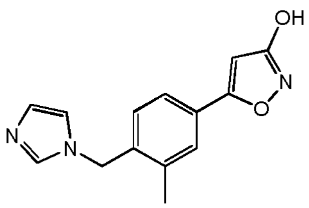
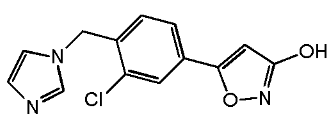

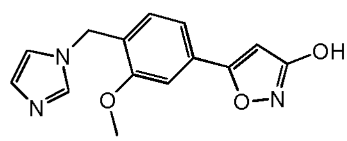
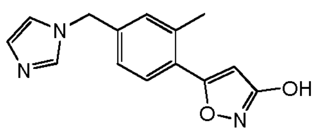
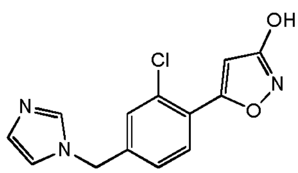

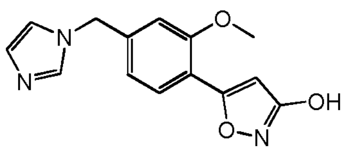
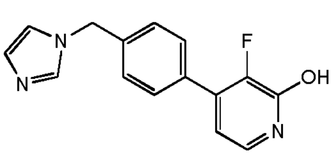
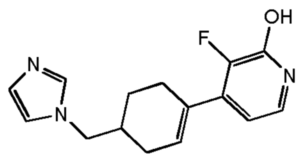

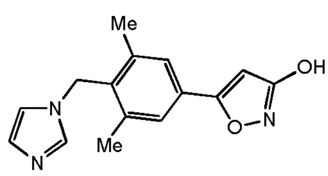
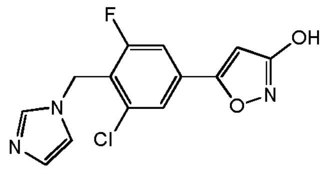
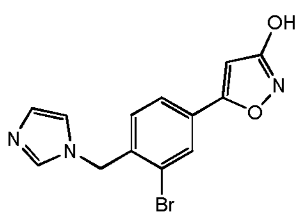

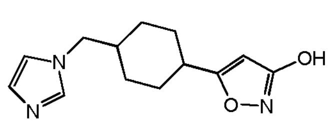
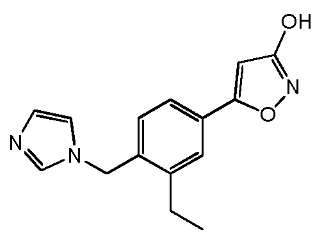
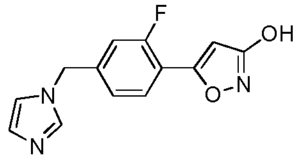

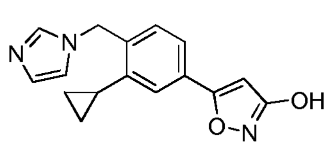 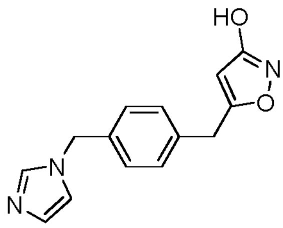 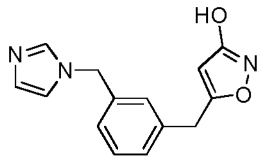

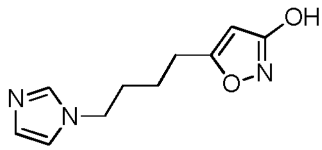.

10. The compound, the pharmaceutically acceptable salt or the tautomer thereof according to claim 1 , which is **characterized in that**, the tautomer of the compound of formula (I) is selected from the group consisting of

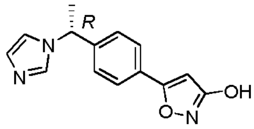 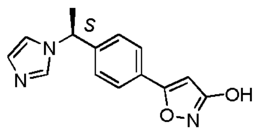 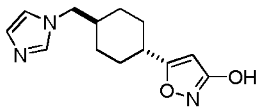 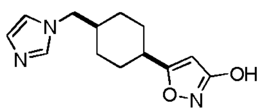.

11. A method of anti-platelet aggregation, comprising administering to a patient in need thereof a therapeutically effective amount of the compound, the pharmaceutically acceptable salt thereof, or the tautomer thereof according to any one of claims 1 to 10.

12. A method of treating ischemic cerebrovascular disease, comprising administering to a patient in need thereof a therapeutically effective amount of the compound, the pharmaceutically acceptable salt thereof, or the tautomer according to any one of claims 1 to 10; wherein, preferably, the ischemic cerebrovascular disease comprises acute cerebral infarction.

| INTERNATIONAL SEARCH REPORT | International application No. PCT/CN2016/079992 |
|---|---|

**A. CLASSIFICATION OF SUBJECT MATTER**

C07D 413/10 (2006.01) i, C07D 233/60 (2006.01) i, C07D 413/06 (2006.01) i, C07D 413/08 (2006.01) i, C07D 413/14 (2006.01) i, C07D 417/10 (2006.01) i, 401/10 (2006.01) i, A61P 9/10 (2006.01) i

According to International Patent Classification (IPC) or to both national classification and IPC

B. FIELDS SEARCHED

Minimum documentation searched (classification system followed by classification symbols)

C07D; A61P

Documentation searched other than minimum documentation to the extent that such documents are included in the fields searched

Electronic data base consulted during the international search (name of data base and, where practicable, search terms used)

DWPI; VEN; CNABS; SIPOABS; CNKI; STN(REG, CAPLUS): imidazolyl, heterocyclyl, phenyl, thromboembolic, cardiotonic

C. DOCUMENTS CONSIDERED TO BE RELEVANT

| Category* | Citation of document, with indication, where appropriate, of the relevant passages | Relevant to claim No. |
|---|---|---|
| X | US 4507298 A (NATTERMANN A & CIE) 26 March 1985 (26.03.1985) see claim 1, embodiment 1 | 1-10 |
| X | US 2009269301 A1 (PROBIODRUG AG) 29 October 2009 (29.10.2009) paragraph [0280], compounds 22, 24, 30, 31, 38, and 48 | 1-3, 5 |
| X | WO 2013049565 A1 (ENDO PHARMACEUTICALS INC.) 04 April 2013 (04.04.2013) page 78, table 1 | 1-5 |
| X | US 4504479 A (NATTERMANN A & CIE) 12 March 1985 (12.03.1985) see embodiment 1, claims 1 and 2 | 1-10 |
| A | US 4643998 A (NATTERMANN A & CIE) 17 February 1987 (17.02.1987) see the whole document | 1-10 |
| A | US 4353905 A (WARNER LAMBERT CO.) 12 October 1982 (12.10.1982) see the whole document | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 26 January 2016 | 26 January 2016 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br>LIU, Yanming<br>Telephone No. (86-10) 62089194 |

Form PCT/ISA /210 (second sheet) (July 2009)

# INTERNATIONAL SEARCH REPORT

International application No. PCT/CN2016/079992

**Box No. II Observations where certain claims were found unsearchable (Continuation of item 2 of first sheet)**

This international search report has not been established in respect of certain claims under Article 17(2)(a) for the following reasons:

1. ☒ Claims Nos.:11-12
because they relate to subject matter not required to be searched by this Authority, namely:
relate to the method of treatment of the human or animal body by therapy (Rule 39. 1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA /210 (continuation of first sheet (2)) (July 2009)

**INTERNATIONAL SEARCH REPORT**
Information on patent family members

International application No.
PCT/CN2016/079992

| Patent Documents referred in the Report | Publication Date | Patent Family | Publication Date |
|---|---|---|---|
| US 4507298 A | 26 March 1985 | EP 0071060 B1 | 10 September 1986 |
| | | IE 53398 B1 | 09 November 1988 |
| | | IE 821632 L | 31 January 1983 |
| | | JPH 0372068 B2 | 15 November 1991 |
| | | JPS 5826883 A | 17 February 1983 |
| | | EP 0071060 A2 | 09 February 1983 |
| | | GR76192B | 03 August 1984 |
| | | DE 3130251 C2 | 08 June 1989 |
| | | DK 343582 A | 01 February 1983 |
| | | EP 0071060 A3 | 21 March 1984 |
| | | ZA 8204883 A | 27.04.1983 |
| | | DE 3130251 A1 | 17 February 1983 |
| US 2009269301 A1 | 29 October 2009 | JP 2013237688 A | 28.11.2013 |
| | | EP 2089383 B1 | 16.09.2015 |
| | | US 8278345 B2 | 02.10.2012 |
| | | WO 2008055945 A1 | 15.05.2008 |
| | | JP 2010509284 A | 25 March 2010 |
| | | JP 5379692 B2 | 25.12.2013 |
| | | EP 2089383 A1 | 19.08.2009 |
| | | JP 5798157 B2 | 21.10.2015 |
| WO 2013049565 A1 | 04 April 2013 | EP 2760854 A1 | 06.08.2014 |
| | | JP 2014531459 A | 27.11.2014 |
| | | CA 2850508 A1 | 04.04.2013 |
| US 4504479 A | 12 March 1985 | DK 343482 A | 01.02.1983 |
| | | EP 0071059 B1 | 24.09.1986 |
| | | DK 157549 B | 22.01.1990 |
| | | ZA 8204882 A | 27.04.1983 |

Form PCT/ISA /210 (patent family annex) (July 2009)

**INTERNATIONAL SEARCH REPORT**
Information on patent family members

International application No.
PCT/CN2016/079992

| Patent Documents referred in the Report | Publication Date | Patent Family | Publication Date |
|---|---|---|---|
| | | JPS 5826885 A | 17.02.1983 |
| | | DK 157549 C | 11.06.1990 |
| | | DE 3130252 C2 | 27.04.1989 |
| | | IE 53469 B1 | 23.11.1988 |
| | | EP 0071059 A2 | 09.02.1983 |
| | | DE 3130252 A1 | 17.02.1983 |
| | | EP 0071059 A3 | 21.03.1984 |
| | | IE 821633 L | 31.01.1983 |
| | | GR76191B | 03.08.1984 |
| US 4643998 A | 17.02.1987 | DK 505983 D0 | 04.11.1983 |
| | | ES 527027 D0 | 01.07.1984 |
| | | PT77616A | 01.12.1983 |
| | | JPH 054391 B2 | 19.01.1993 |
| | | PT77616B | 19.03.1986 |
| | | IE 832484 L | 06.05.1984 |
| | | DK 505983 A | 07.05.1984 |
| | | EP 0112987 A1 | 11.07.1984 |
| | | DE 3241102 A1 | 10.05.1984 |
| | | IE 56127 B1 | 24.04.1991 |
| | | GR78945B | 02.10.1984 |
| | | DK 156660 B | 18.09.1989 |
| | | JPS 59108781 A | 23.06.1984 |
| | | DK 156660 C | 05.03.1990 |
| | | ZA 8307976 A | 27.06.1984 |
| | | EP 0112987 B1 | 01.04.1987 |
| | | ES 8406472 A1 | 01.11.1984 |
| US 4353905 A | 12.10.1982 | ZA 8206767 A | 27.07.1983 |
| | | JPS5874679A | 06.05.1983 |

Form PCT/ISA /210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201510200711


### Non-patent literature cited in the description


- **BERGE et al.** Pharmaceutical Salts. *Journal of Pharmaceutical Science,* 1977, vol. 66, 1-19
- **MAEHR.** *J. Chem. Ed.,* 1985, vol. 62, 114-120
- Remington: The Science and Practice of Pharmacy. Lippincott, Williams & Wilkins, 2005